# EUROPÄISCHE PATENTSCHRIFT

(11) **EP 3 891 161 B1**
(45) Veröffentlichungstag und Bekanntmachung des Hinweises auf die Patenterteilung: **17.06.2026**
(21) Anmeldenummer: 19812757.3
(22) Anmeldetag: 27.11.2019
(51) Int. Cl.: C07H 3/02, C07H 1/06, B01D 9/02, C13B 30/00

(54) **KONTINUIERLICHES VERFAHREN ZUR GEWINNUNG EINES KRISTALLINEN MONOSACCHARIDES UND VORRICHTUNG ZUR KONTINUIERLICHEN KRISTALLISIERUNG**
CONTINUOUS PROCESS FOR PRODUCING CRYSTALLINE MONOSACCHARIDES AND APPARATUS FOR CONTINUOUS CRYSTALLIZATION
PROCÉDÉ CONTINU POUR LA PRÉPARATION DE MONOSACCHARIDES CRISTALLINS ET APPAREIL DE CRISTALLISATION CONTINUE

(30) Priorität: 06.12.2018 DE 102018131131
(43) Veröffentlichungstag der Anmeldung: 13.10.2021
(73) Patentinhaber: BMA Braunschweigische Maschinenbauanstalt GmbH, 38122 Braunschweig (DE)
(72) Erfinder: LÖHN, Mirko, 38154 Königslutter (DE); FERSTERRA, Holger, 38116 Braunschweig (DE)
(74) Vertreter: Meissner Bolte Partnerschaft mbB
(86) Internationale Anmeldenummer: PCT/EP2019/082685
(87) Internationale Veröffentlichungsnummer: WO 2020/114850

(56) Entgegenhaltungen:
- EP-A1- 1 310 504
- EP-A1- 3 210 478
- WO-A2-2018/081557
- DE-A1- 4 041 317

## Beschreibung

Die Erfindung betrifft ein kontinuierliches Verfahren zur Gewinnung eines kristallinen Monosaccharides und eine Vorrichtung zur Gewinnung eines kristallinen Monosaccharides, insbesondere zur Durchführung des erfindungsgemäßen kontinuierlichen Verfahrens.

Zur Kristallisation von Sacchariden werden Verdampfungskristallisatoren und Kühlungskristallisatoren eingesetzt, deren Aufbau und Funktionsweise dem Fachmann bekannt sind. Um spontane Kristallbildung zu verhindern, wird zur Initiierung des Kristallisatiosprozesses einer kristallfreien konzentrierten Lösung, die den Zucker enthält, Saatkristalle oder ein Kristallisationsmagma, das Saatkristalle enthält, zugegeben, so dass ein Kristallwachstum unter kontrollierten Bedingungen in einem Kristallisator ablaufen kann.

Allulose (D-Psicose) ist ein Monosaccharid aus der Gruppe der Ketohexosen, welches durch die Entwicklung neuer Verfahren in größeren Mengen herstellbar geworden ist (Takeshita et al., Journal of Bioscience and Bioengineering Vol. 90, Nr. 4, S. 453 bis 455, 2000; Koreanische Patentanmeldung Nr. 10-2009-0118465, CJ Cheiljedang Corp. Korea).

Die EP 1310504 A1 offenbart ein Verfahren zur Herstellung von Tagatose-Kristallen aus einem wässrigen System.

Die WO 2018/081557 A2 offenbart ein Verfahren zur Herstellung von Allulosekristallen.

Die DE 4041317 A1 offenbart ein Verfahren zum Kristallisieren kristallwasserfreier Fruktose aus Wasser.

Die Kristallisation von Allulose im industriellen Maßstab wird in der Literatur durch eine Patentanmeldung bzw. Patenterteilung beschrieben (PCT/KR2015/009449 bzw. EP 3210478 A1, beide CJ Cheiljedang Corp., Korea). In der Druckschrift wird beschrieben, dass D-Psicose nach der Aufreinigung und Konzentrierung durch die Anwendung der Kühlungskristallisation aus der flüssigen Phase in den kristallinen Zustand überführt wird, wobei die Lösung nach der Konzentrierung mittels Wärmetauscher auf 30 bis 40 °C gekühlt und mit Saatkristallen versetzt wird. Die Herstellung der Saatkristalle zur Initiierung der Allulosekristallisation wird nicht beschrieben. Wenn die Kristallwachstumsgeschwindigkeit nachlässt (bei Erreichen des Gleichgewichtszustandes) wird zur Kristallsuspension 1 bis 2 mal pro Stunde eine definierte Menge der auf 30 bis 40 °C gekühlten konzentrierten Alluloselösung zugegeben. Diese Prozedur wird wiederholt, bis der Kristallisator sein maximales Arbeitsvolumen erreicht hat.

Durch die diskontinuierliche Herstellungsweise ist eine industrielle Produktion kristalliner Allulose aufwendig und unwirtschaftlich. Auch für andere Monosaccharide besteht der Bedarf, eine kontinuierliche Herstellung der kristallinen Form zu ermöglichen oder bestehende Verfahren und Vorrichtungen zu verbessern. Eine besondere Schwierigkeit bei der industriellen Herstellung kristalliner Zucker besteht darin, das Kristallwachstum zu steuern. Auf der einen Seite wird eine möglichst hohe Ausbeute angestrebt. Andererseits führen Bedingungen, die eine hohe Ausbeute ermöglichen, nicht unbedingt zu einem Verfahren, das als großtechnisches, insbesondere kontinuierliches, Verfahren geeignet ist. Das Kristallwachstum kann von zahlreichen Faktoren beeinflusst werden, beispielsweise durch die Kristallisationstemperatur und -art, die Durchmischung der Kristallsuspension sowie durch die Art des Zuckers. Diese Faktoren können somit die Ausbeute, die Form und die Größe der Zuckerkristalle beeinflussen. Wiederum können sich Form und die Größe der Kristalle sowie deren Konzentration in der Kristallsuspension auf das Fließverhalten der Kristallsuspension in einer Anlage auswirken. Gelingt es nicht, das Kristallwachstum genau zu steuern, ist ein großtechnisches und insbesondere kontinuierliches Verfahren wegen der mangelhaften Bearbeitbarkeit der Kristallsuspensionen nicht denkbar. Auch mit Blick auf weitere Verfahrensschritte wird im Allgemeinen eine geringe Partikelgrößenverteilung angestrebt. Eine weitere Schwierigkeit besteht in einem kontinuierlichen Herstellungsverfahren darin, kontinuierlich eine ausreichend große Masse an Kristallisationsmagma zur Beimpfung einer Kristallisationslösung herzustellen. Denn während eines Kristallisationsschrittes ist die technische erreichbare Entzuckerung der Lösung durch den Kristallgehalt in einer Kristallsuspension begrenzt. Eine Limitation der verfügbaren Masse an Kristallisationsmagma rührt daher, dass die Herstellungsverfahren für Kristallisationsmagma meist diskontinuierlich durchgeführt werden.

Daher besteht eine Aufgabe der vorliegenden Erfindung darin, ein kontinuierliches Verfahren zur Gewinnung eines kristallinen Monosaccharides und eine Vorrichtung zur Gewinnung eines kristallinen Monosaccharides, insbesondere zur Durchführung des kontinuierlichen erfindungsgemäßen Verfahrens, bereitzustellen.

Auch die kontinuierliche Bereitstellung einer ausreichend großen Menge an Kristallisationsmagma zur Beimpfung der Kristallisationslösung soll durch das Verfahren bzw. die Vorrichtung ermöglicht werden, damit das gesamte Verfahren kontinuierlich durchgeführt werden kann.

Ferner muss das Kristallwachstum in dem Verfahren bzw. der Vorrichtung durch die gegebenen Bedingungen kontrolliert sein und zu handhabbaren, z.B. rühr- und homogenisierbaren, Gemischen führen.

Eine weitere Aufgabe ist es, mit dem Verfahren bzw. der Vorrichtung ein kontinuierliches Verfahren mit hoher Effizienz und Ausbeute in großen Mengen zu ermöglichen.

Erfindungsgemäß wird diese Aufgabe durch ein Verfahren nach Anspruch 1 gelöst. Mit Blick auf die Vorrichtung wird diese Aufgabe durch den Gegenstand des Anspruches 15 gelöst.

Bevorzugt wird zumindest eine dieser Aufgaben durch das erfindungsgemäße kontinuierliche Verfahren zur Gewinnung eines kristallinen Monosaccharides gelöst, wobei das kontinuierliche Verfahren bevorzugt eine kontinuierliche Kristallisation des Monosaccharides in einem Hauptkristallisator, ein Abtrennen von Kristallen des Monosaccharides aus einer Kristallsuspension (einem Kristallisationsgemisch), um kristallines Monosaccharid zu gewinnen, die kontinuierliche Bildung einer Masse an Kristallisationsmagma für den Hauptkristallisator in einer Kaskade (aus Vorkristallisatoren) und das kontinuierliche Zuführen einer Lösung, die das Monosaccharid enthält, und einer Masse eines Kristallisationsmagmas aus dem mindestens einem Vorkristallisator der letzten Stufe der Kaskade in den Hauptkristallisator, um die Kristallsuspension bereitzustellen, umfasst. Während der kontinuierlichen Kristallisation des Monosaccharides in einem Hauptkristallisator wird in dem Hauptkristallisator an einer Kristallsuspension kontinuierlich eine Verdampfungs- und/oder Kühlungskristallisation durchgeführt, um in der Kristallsuspension Kristalle des Monosaccharides wachsen zu lassen. Die kontinuierliche Bildung einer Masse an Kristallisationsmagma für den Hauptkristallisator erfolgt in einer Kaskade, wobei die Kaskade in Reihe geschaltet mindestens eine erste und eine letzte Stufe umfasst und jede Stufe mindestens einen Vorkristallisator umfasst, wobei in dem mindestens einem Vorkristallisator der ersten Stufe Lösung mit Monosaccharid durch Monosaccharid-Saatkristalle beimpft wird, um ein (sogenanntes) Prä-Kristallisationsmagma zu erhalten, und aus dem Prä-Kristallisationsmagma mittels Kühlungskristallisation und/oder Verdampfungskristallisation eine Masse an Kristallisationsmagma für die nachgeschaltete Stufe gebildet wird, und wobei in den mindestens einen Vorkristallisator der letzten Stufe Lösung mit Monosaccharid und eine Masse an Kristallisationsmagma aus der vorgeschalteten Stufe zugeführt wird, um ein Prä-Kristallisationsmagma zu erhalten, und in dem mindestens einem Vorkristallisator der letzten Stufe aus dem Prä-Kristallisationsmagma mittels Kühlungskristallisation und/oder Verdampfungskristallisation eine Masse an Kristallisationsmagma für den Hauptkristallisator gebildet wird.

Durch dieses kontinuierliche Verfahren wird die effiziente Gewinnung eines kristallinen Monosaccharides möglich und wirtschaftlich.

Insbesondere gelingt die kontinuierliche Bereitstellung von Kristallisationsmagma in ausreichenden Massen, so dass das gesamte Verfahren kontinuierlich durchgeführt werden kann.

Ferner ist das Kristallwachstum unter den gegebenen Bedingungen kontrollierbar und führt zu aufeinander abgestimmten Kristallsuspensionen, so dass das Verfahren kontinuierlich in einer großtechnischen Anlage durchgeführt werden kann, so dass der apparative Aufwand minimiert ist. Durch die mehrstufige Kristallisationsmagmaerzeugung kann gezielt Einfluss auf die Partikelgrößenverteilung genommen werden. In der anschließenden kontinuierlichen Kristallisation im Hauptkristallisator kann dadurch das Kristallgrößenwachstum sehr exakt auf die Zielkristallgröße eingestellt werden. Ferner weist das kontinuierliche Verfahren eine hohe Effizienz und Ausbeute auf, wie nachfolgend erläutert.

Die kontinuierliche Kristallisation des Monosaccharides in dem Hauptkristallisator erfolgt durch Kühlen oder alternativ durch Verdampfen oder auch durch eine Kombination der beiden Verfahren.

Eine Verdampfungskristallisation kann unter atmosphärischen Druck oder vorzugsweise darunter durchgeführt werden.

Aufbau und Funktionsweise von Verdampfungskristallisatoren und Kühlungskristallisatoren sind dem Fachmann bekannt. Auch die Durchführung einer Verdampfungskristallisation und/oder einer Kühlungskristallisation, jeweils kontinuierlich oder diskontinuierlich, sind dem Fachmann bekannt. Die kontinuierliche Kristallisation des Monosaccharides in einem Hauptkristallisator kann im Falle der Anwendung der Kühlungskristallisation beispielsweise mit einem BMA OVC (Vertikaler Kühlungskristallisator mit oszillierenden Kühlrohrbündeln, "oscillating vertical cooling crystallizer") durchgeführt werden. Die kontinuierliche Kristallisation des Monosaccharides in einem Hauptkristallisator kann im Falle der Anwendung der Verdampfungskristallisation mit einem BMA VKT (Verdampfungs-Kristallisations-Turm) durchgeführt werden. Als Vorteile ergeben sich aus einer kontinuierlichen Kristallisation bessere Raum-Zeit-Ausbeuten, eine Verminderung der Rüstzeiten für Reinigung, Befüllung und Entleerung, die Erzielung höherer Durchsatzmengen bei geringerem Platzbedarf. Insgesamt ist die Produktivität deutlich erhöht, während eine batchweise Produktion deutlich arbeitsaufwendiger ist.

Die Lösungen, Kristallsuspension, das Prä-Kristallisationsmagma, das Kristallisationsmagma, die Saatsuspension etc. weisen in der vorliegenden Patentanmeldung als Lösungsmittel bevorzugt Wasser auf. Denkbar sind aber auch anderen Lösungsmittel, insbesondere Alkohole und Mischungen davon mit Wasser.

Eine "Lösung, die das Monosaccharid enthält," beschreibt eine Lösung, die das Monosaccharid umfasst und dem Hauptkristallisator zugeführt wird.

Eine "Lösung mit Monosaccharid" beschreibt eine Lösung, die das Monosaccharid umfasst und einem Vorkristallisator zugeführt wird.

In bevorzugten Ausführungsformen unterscheidet sich die "Lösung, die das Monosaccharid enthält" nicht von der "Lösung mit Monosaccharid". Mit anderen Worten ist in bevorzugten Ausführungsformen die "Lösung mit Monosaccharid" die "Lösung, die das Monosaccharid enthält".

Die Lösung mit Monosaccharid und die Lösung, die das Monosaccharid enthält, können dieselben Bestandteile und Eigenschaften aufweisen, d.h. identisch sein, oder verschiedene Bestandteile und Eigenschaften aufweisen, d.h. unterschiedlich sein.

Die Lösung mit Monosaccharid und die Masse an Kristallisationsmagma aus der vorgeschalteten Stufe kann in dem mindestens einen Vorkristallisator jeder Stufe oder zuvor vereint werden. Die Lösung, die das Monosaccharid enthält, und die Masse an Kristallisationsmagma kann in dem Hauptkristallisator oder zuvor vereint werden. Das Beimpfen der Lösung mit Monosaccharid durch Monosaccharid-Saatkristalle kann in dem mindestens einen Vorkristallisator der ersten Stufe erfolgen und/oder in einer vorgeschalteten Beimpfvorrichtung. Mit anderen Worten kann der mindestens eine Vorkristallisator der ersten Stufe eine Beimpfvorrichtung umfassen.

Die Kühlungs- oder Verdampfungskristallisation in einer Stufe der Kaskade kann jederzeit bei Erreichen bestimmter Qualitätsmerkmale des Kristallisationsmagmas (z.B. Form, Größe, Größenverteilung, Viskosität) in Abhängigkeit des Monosaccharids beendet werden. Da die Ausbeute (gebildete Kristallmasse bezogen auf die Ausgangsmasse der kristallbildenden Substanz in der Lösung/den Lösungen bzw. Abbau der Trockensubstanz in der flüssigen Phase) im Schritt der kontinuierlichen Kristallisation im Hauptkristallisator bestimmt wird, sind die Ausbeuten innerhalb der Kaskade für das Gesamtverfahren unerheblich.

Um spontane Kristallbildung zu verhindern, wird zur Initiierung des Kristallisationsprozesses und zur Erzeugung einer definierten Kristallgröße in dem mindestens einem Vorkristallisator der ersten Stufe Lösung mit Monosaccharid mit Monosaccharid-Saatkristallen beimpft, die in der Lösung suspendiert werden. Die Zugabe der Monosaccharid-Saatkristalle kann in trockener Form erfolgen oder durch die Zugabe in Form einer Saatsuspension, einer sogenannten Slurry, in der die Monosaccharid-Saatkristalle in einem Suspensionsmittel suspendiert sind. Die Saatsuspension wird aus kristallinem Monosaccharid hoher Reinheit (> 99%) durch Zerkleinern, vorzugsweise durch Vermahlen von kristallinem Monosaccharid mit Isopropanol oder durch Vermahlen einer übersättigten, wässrigen Monosaccharidlösung hergestellt, so dass die suspendierten Partikel vorzugsweise eine Größe von 10 bis 20 µm haben.

Zur Erzeugung von Prä-Kristallisationsmagma bzw. einer Masse an Kristallisationsmagma in dem mindestens einem Vorkristallisator der ersten Stufe kann entsprechend bekannter Berechnungsgleichungen (z.B.: V.Gnielinski, A.Mersmann, F.Thurner: "Verdampfung, Kristallisation, Trocknung", Springer Fachmedien Wiesbaden GmbH 1993) die notwendige Kristallkonzentration (Kristallanzahl in der zu beimpfenden Menge) in Abhängigkeit der Kristallgröße der Saatsuspension, der gewünschten Endgröße der Kristalle, des zu erzielenden Kristallgehaltes und der Kristallform berechnet werden.

Das Prä-Kristallisationsmagma und das Kristallisationsmagma enthalten Kristalle des Monosaccharides.

Bevorzugt ist das Prä-Kristallisationsmagma eine Suspension mit Kristallen des Monosaccharides und wird aus einer Lösung mit Monosaccharid und einer Masse an Kristallisationsmagma oder Monosaccharid-Saatkristallen gebildet.

Bevorzugt wird aus dem Prä-Kristallisationsmagma in einem Vorkristallisator durch Kristallisation ein Kristallisationsmagma gebildet. Das Kristallisationsmagma wird bevorzugt zur Beimpfung einer Lösung mit Monosaccharid in einem Vorkristallisator und/oder zur Beimpfung einer Lösung, die das Monosaccharid enthält, in dem Hauptkristallisator verwendet.

Das Prä-Kristallisationsmagma und das Kristallisationsmagma enthalten Kristalle des Monosaccharides.

Bevorzugt beschreibt der Begriff "Kristallisator" eine Vorrichtung, insbesondere zur Durchführung eines Kristallisationsprozesses.

Bevorzugt ist ein Vorkristallisator ein Kristallisator, insbesondere zur Erzeugung von Kristallisationsmagma.

Bevorzugt beschreibt der Begriff "Hauptkristallisator" eine Vorrichtung, in der ein Großteil des kristallinen Monosaccharides gebildet wird.

Der Begriff "Verweilzeit" beschreibt die hydraulische Verweilzeit, die sich aus dem Volumen eines Kristallisators geteilt durch den Volumenstrom ergibt.

Bevorzugte Ausführungsformen werden in den Unteransprüchen angegeben.

Verschiedene Ausführungsformen der Erfindung können miteinander kombiniert werden, sofern sich aus dem Kontext nicht etwas Anderes ergibt.

In einer bevorzugten Ausführungsform umfasst jede Stufe einen einzigen Vorkristallisator und in dem Vorkristallisator jeder Stufe wird aus dem Prä-Kristallisationsmagma mittels Verdampfungskristallisation kontinuierlich eine Masse an Kristallisationsmagma gebildet.

Diese Ausführungsform ermöglicht eine einfachere Ausgestaltung des Verfahrens. Eine Verdampfungskristallisation kann kontinuierlich durchgeführt werden, wodurch kontinuierlich eine Masse an Kristallisationsmagma gebildet werden kann. Durch die Kaskade an Vorkristallisatoren kann eine große Masse an Kristallisationsmagma gebildet werden.

In einer weiteren bevorzugten Ausführungsform umfasst jede Stufe zwei bis drei Vorkristallisatoren und in den Vorkristallisatoren jeder Stufe wird aus dem Prä-Kristallisationsmagma diskontinuierlich mittels Kühlungskristallisation und/oder kontinuierlich mittels Verdampfungskristallisation eine Masse an Kristallisationsmagma gebildet, wobei die Masse an Kristallisationsmagma, die dem Hauptkristallisator kontinuierlich zugeführt wird, bei diskontinuierlicher Bildung mittels Kühlungskristallisation alternierend aus den Vorkristallisatoren der letzten Stufe zugeführt wird.

Wird das Kristallisationsmagma diskontinuierlich mittels Kühlungskristallisation in nur einem Vorkristallisator pro Stufe gebildet, so ist eine kontinuierliche Zuführung und auch einer ausreichenden Menge in den Hauptkristallisator für ein kontinuierliches Kristallisationsverfahren nicht gegeben. Dieses Problem kann durch diese zuvor beschriebene Ausführungsform überwunden werden. Da jede Stufe mehrere Vorkristallisatoren aufweist, kann auch bei Verwendung einer diskontinuierlichen Kühlungskristallisation in der letzten Stufe abwechselnd aus mehreren Vorkristallisatoren Kristallisationsmagma kontinuierlich in den Hauptkristallisator zugeführt werden. Mit anderen Worten sind die Vorkristallisatoren der Stufen so miteinander verschaltet, dass eine kontinuierliche Zuführung des Kristallisationsmagmas aus der letzten Stufe der Kaskade zum kontinuierlich arbeitenden Hauptkristallisator gewährleistet wird.

Zu beachten ist dabei, dass nicht jede Stufe die gleiche Anzahl an Vorkristallisatoren aufweisen muss. Ferner können die Vorkristallisatoren der Stufen in vielfältiger Weise untereinander verbunden sein.

Die Verdampfungskristallisation kommt insbesondere dann zur Anwendung, wenn die Rahmenbedingungen für eine Verdampfungskristallisation des Monosaccharids gegeben sind. Diese sind von der Temperaturempfindlichkeit der Lösung und von der Löslichkeit des Produktes abhängig. Nimmt die Löslichkeit mit steigender Temperatur nur mäßig oder sehr wenig zu, so wird bevorzugt die Verdampfungskristallisation zur Anwendung kommen, dann oft auch unter Vakuum betrieben.

Nimmt die Löslichkeit mit steigender Temperatur stark zu, so ist das bevorzugte Kristallisationsverfahren die Kühlungskristallisation.

Bevorzugt ist in bestimmten Ausführungsformen, dass die erste Stufe ein bis zwei, bevorzugt zwei, Vorkristallisatoren, die letzte Stufe zwei bis vier, bevorzugt drei, Vorkristallisatoren und eine weitere Stufe zwei bis vier, bevorzugt zwei, Vorkristallisatoren aufweist, wobei in den Vorkristallisatoren jeder Stufe aus dem Prä-Kristallisationsmagma diskontinuierlich mittels Kühlungskristallisation eine Masse an Kristallisationsmagma gebildet wird und wobei die Masse an Kristallisationsmagma, die dem Hauptkristallisator kontinuierlich zugeführt wird, alternierend aus den Vorkristallisatoren der letzten Stufe zugeführt wird.

In einer bestimmten Ausführungsform, in welcher mindestens eine Stufe mehr als einen Vorkristallisator umfasst, bilden die Vorkristallisatoren derselben Stufe jeweils die gleiche Masse an Kristallisationsmagma.

Dies ermöglicht, dass abwechselnd aus mehreren Vorkristallisatoren eine große Masse an Kristallisationsmagma kontinuierlich in den Hauptkristallisator zugeführt werden kann.

In einer bevorzugten Ausführungsform übersteigt die Masse an Kristallisationsmagma, die in einem Vorkristallisator einer Stufe gebildet wird, die Masse an Kristallisationsmagma, die in einem Vorkristallisator der vorgeschalteten Stufe gebildet wird, um den Faktor 2 bis 12, bevorzugt 4 bis 7.

Somit kann mit jeder Stufe die Masse an Kristallisationsmagma beträchtlich gesteigert werden, so dass genug Kristallisationsmagma für ein kontinuierliches Zuführen einer Masse eines Kristallisationsmagmas in den Hauptkristallisator und damit eine kontinuierliche Kristallisation des Monosaccharides in dem Hauptkristallisator sichergestellt werden kann. Dabei kann der Faktor nicht beliebig gesteigert werden, da in dem Prä-Kristallisationsmagma in den Vorkristallisatoren eine bestimmte Konzentration an zugeführten Kristallisationsmagma vorliegen muss, um ein effizientes und berechenbares Kristallwachstum zu erreichen.

In einer bevorzugten Ausführungsform umfasst die Kaskade zwischen der ersten und der letzten Stufe eine bis acht, bevorzugt eine bis drei, am bevorzugtesten eine, in Reihe geschaltete weitere Stufe bzw. Stufen, wobei die weitere Stufe bzw. weiteren Stufen jeweils mindestens einen Vorkristallisator aufweist bzw. aufweisen, in welchem bzw. welchen Lösung mit Monosaccharid und eine Masse an Kristallisationsmagma aus der vorgeschalteten Stufe zugeführt wird, um Prä-Kristallisationsmagma zu erhalten, und wobei in dem mindestens einen Vorkristallisator jeder weiteren Stufe aus dem Prä-Kristallisationsmagma diskontinuierlich mittels Kühlungskristallisation und/oder kontinuierlich mittels Verdampfungskristallisation eine Masse an Kristallisationsmagma für die nachgeschaltete Stufe gebildet wird.

Durch weitere Stufen kann die Masse an Kristallisationsmagma, die aus der letzten Stufe der Kaskade in den Hauptkristallisator geführt wird, weiter gesteigert werden.

Somit kann mit jeder zusätzlichen Stufe die Masse an Kristallisationsmagma beträchtlich gesteigert werden, so dass genug Kristallisationsmagma für ein kontinuierliches Zuführen einer Masse eines Kristallisationsmagmas in den Hauptkristallisator und damit eine kontinuierliche Kristallisation des Monosaccharides in dem Hauptkristallisator sichergestellt werden kann. Die bevorzugte Anzahl an Stufen ermöglicht ein berechenbares Kristallwachstum, welches den chemischphysikalischen Eigenschaften des Monosaccharids und der gewünschten Produktionsmenge angepasst ist.

In bestimmten Ausführungsformen sind die Stufen bzw. der mindestens eine Vorkristallisator jeder Stufe derart miteinander verbunden, dass es möglich ist, einzelne weitere Stufen auszulassen.

Dies ist insbesondere vorteilhaft, um die Anlage kontinuierlich warten oder reinigen zu können. Ferner ist dies vorteilhaft, um geringere Mengen an Kristallisationsmagma zu erzeugen, falls für die Hauptkristallisation weniger Kristallisationsmagma benötigt wird.

In einer bevorzugten Ausführungsform weisen die Monosaccharid-Saatkristalle einen mittleren Durchmesser von 5 bis 50 µm, bevorzugt 10 bis 20 µm, auf. Es wurde herausgefunden, dass ein kontinuierliches Verfahren zur Gewinnung eines kristallinen Monosaccharides insbesondere dann gut durchführbar ist, wenn die Monosaccharid-Saatkristalle diesen Durchmesser aufweisen. Durch die Herstellung des Kristallisationsmagmas in der Kaskade erhöht sich die mittlere Partikelgröße von Stufe zu Stufe. Bei einer Ausgangsgröße wie oben genannt erfolgt das Kristallwachstum in Kaskade und Hauptkristallisator derart, dass im Hauptkristallisator Kristalle mit einer guten Ausbeute in gewünschter Größe erhalten werden.

In einer bestimmten bevorzugten Ausführungsform wird ein Temperaturgradient der Kristallsuspension über die Länge des Hauptkristallisators von 70 bis 15 °C und bevorzugt von 45 bis 25 °C eingestellt.

In bestimmten Ausführungsformen wird in dem Hauptkristallisator an einer Kristallsuspension kontinuierlich eine Kühlungskristallisation durchgeführt, wobei die Kristallsuspension in dem Hauptkristallisator von bevorzugt 70 - 30 °C bevorzugt bis auf 35 - 15 °C abgekühlt wird.

In bestimmten ähnlichen Ausführungsformen wird in dem Hauptkristallisator an einer Kristallsuspension kontinuierlich eine Kühlungskristallisation durchgeführt, wobei die Kristallsuspension in dem Hauptkristallisator von bevorzugt 70 - 33 °C bevorzugt bis auf 32 - 15 °C abgekühlt wird.

In bestimmten Ausführungsformen wird im kontinuierlich arbeitenden Hauptkristallisator bei der Kühlungskristallisation mit einem Temperaturgradienten (Temperaturprofil) in Abhängigkeit vom Monosaccharid von oben nach unten gearbeitet. In diesen Ausführungsformen beträgt die Temperatur der Kristallsuspension im Bereich der Zuführung der Lösung, die das Monosaccharid enthält, und des Kristallisationsmagmas (oben) bevorzugt von 70 - 30 °C und am Austragsbereich der Kristallsuspension (unten) bevorzugt von 35 - 15 °C.

Bei diesen Temperaturprofilen erfolgt eine Kristallbildung in gewünschter Größe und Ausbeute. Gleichzeitig wird die Kristallsuspension nicht zu zähflüssig, was die weitere Verarbeitung erschweren würde.

In einer besonders bevorzugten Ausführungsform wird in dem Hauptkristallisator an einer Kristallsuspension kontinuierlich eine Kühlungskristallisation durchgeführt, wobei die Kristallsuspension in dem Hauptkristallisator von bevorzugt 45 - 35 °C bevorzugt bis auf 30 - 20 °C abgekühlt wird. Diese Ausführungsform ist insbesondere bevorzugt, wenn es sich um ein kontinuierliches Verfahren zur Gewinnung kristalliner Allulose handelt.

Bei diesem Temperaturprofil erfolgt eine Kristallbildung der Allulose in gewünschter Größe und Ausbeute. Gleichzeitig wird die Kristallsuspension der Allulose nicht zu zähflüssig, was die weitere Verarbeitung erschweren würde.

In einer bevorzugten Ausführungsform beträgt die Verweildauer der Kristallsuspension in dem Hauptkristallisator 30 bis 70 Stunden.

Es wurde herausgefunden, dass bei dieser Verweilzeit eine Kristallbildung in gewünschter Größe und Ausbeute erfolgt.

In einer bevorzugten Ausführungsform wird der Inhalt jedes Vorkristallisators, bevorzugt ein oder mehrere Lösungen, Suspensionen, Prä-Kristallisationsmagma und/oder Kristallisationsmagma, von einem Rührer mit einem spezifischen Leistungseintrag von 0,1 bis 4 kW/m³, bevorzugt 0,5 bis 2,0 kW/m³, angetrieben. Durch den Rührprozess wird die kristalline Phase bzw. das Kristallisationsmagma gleichmäßig in der flüssigen Phase bzw. der Lösung mit Monosaccharid verteilt, wodurch der Stofftransport begünstigt und die Zunahme der Kristallmasse pro Zeiteinheit erhöht wird. Weiterhin kommt es zu einer Homogenisierung der Kristalle in den Vorkristallisatoren.

Der Rührertyp, die Rührerform und der spezifische Energieeintrag über den Rührer muss sich in den einzelnen Stufen der Kaskade an den spezifischen Viskositäten orientieren. Bei niedrigen Viskositäten (< 0,5 Pas) wird zum Suspendieren der Kristalle in der flüssigen Phase vorzugsweise ein Schrägblatt- Schaufel- oder Propellerrührer eingesetzt. Im mittleren Viskositätsbereich (0,5 bis 5,0 Pas) kommt vorzugsweise ein Intermig- Kreuzbalken- oder Blattrührer zum Einsatz. Im hohen Viskositäsbereich (> 5,0 Pas) werden vorzugsweise Anker- und Wendelrührer eingesetzt.

Es wurde überraschender Weise gefunden, dass bei der Kristallisation von Allulose durch die Erhöhung des spezifischen Energieeintrages des Rührers, ein fokussiertes Kristallwachstum in Längsrichtung unterdrückt werden konnte, wodurch das Verhältnis von Durchmesser zu Länge von z.B. 1:10 auf die Hälfte reduziert werden konnte. Bei Kristallen, die zu einem Längenwachstum neigen (Stäbchen, Nadeln) kann also durch den spezifischen Energieeintrag über den Rührer Einfluss auf die Morphologie der Kristalle genommen werden. Wird der spezifische Leistungseintrag des Rührwerks von z.B. 0,5 kW/m³ auf z.B. 2,0 kW/m³ erhöht, konnte beobachtet werden, dass dadurch das Wachstumsverhalten der Kristalle (Längenwachstum) beeinflusst werden kann, so dass sich das Längenwachstum vermindern ließ. Die Allulose weist im Gegensatz zur Saccharose ein viel stärkeres Längenwachstum auf. Insofern weisen die Rührer Vorteile auf, die aus herkömmlichen Anlagen für Saccharose nicht bekannt sind. Durch den spezifischen Energieeintrag solcher Rührer wird gezielt Einfluss auf das Kristallwachstum genommen.

Denkbar ist auch, dass in jedem Vorkristallisator mehrerer Rührer verwendet werden, um denselben technischen Effekt zu erreichen.

Bevorzugt wird dem Hauptkristallisator die Lösung, die das Monosaccharid enthält, und eine Masse an Kristallisationsmagma in einem Massenverhältnis von 1:5 bis 1:20, bevorzugt 1:7 bis 1:11, zugeführt.

Somit kann in dem Hauptkristallisator effizient in großen Mengen in relativ kurzer Zeit mit hoher Ausbeute kristallines Monosaccharid gebildet werden.

Bevorzugt weist die Lösung mit Monosaccharid für die Vorkristallisatoren eine Übersättigung von 0 bis 60 % auf. Mit anderen Worten wird das Kristallisationsmagma aus einer vorgeschalteten Stufe in die nachgeschaltete Stufe bereitgestellt und mit frischer, insbesondere kristallfreier Lösung mit Monosaccharid mit einer Übersättigung von 0 bis 60 % innerhalb des metastabilen Bereiches vermischt.

Dadurch wird in jeder Stufe der Kaskade eine konstant hohe Triebkraft für das Kristallwachstum erzeugt.

Bevorzugt werden die Verweilzeiten in den jeweiligen Stufen durch die jeweils vorherrschende Übersättigung bestimmt (Gleichgewichtszustand). Die Kontrolle des Prozesses erfolgt über die Bestimmung des Trockensubstanzgehaltes der flüssigen Phase bzw. des Kristallisationsmagmas, beispielsweise durch Bestimmung des Brechungsindexes, durch radiometrische Dichtemessung oder Mikrowellenmessung. Alternativ können auch optische Verfahren zur Erfassung unerwünschter Neukristallbildung und damit zur Prozessoptimierung eingesetzt werden.

Durch das erfindungsgemäße Verfahren entsteht am Ende der Kaskade ein Kristallisationsmagma mit einer definierten Anzahl von Kristallen gewünschter Korngröße und Korngrößenverteilung, das als Kristallisationsmagma für ein kontrolliertes Kristallwachstum in dem kontinuierlich arbeitenden Hauptkristallisator verwendet werden kann.

Bevorzugt ist die Lösung, die das Monosaccharid enthält, bei Zuführung in den Hauptkristallisator übersättigt.

Somit kann in dem Hauptkristallisator effizient in großen Mengen in kurzer Zeit mit hoher Ausbeute kristallines Monosaccharid gebildet werden.

Bevorzugt weist die Masse an Kristallisationsmagma bei Zuführung in den Hauptkristallisator einen Kristallgehalt von 1 bis 5 % (Gew.-%) und/oder einen mittleren Partikeldurchmesser von 50 bis 150 µm auf.

Somit kann in dem Hauptkristallisator effizient in großen Mengen in kurzer Zeit mit hoher Ausbeute kristallines Monosaccharid gebildet werden. Gleichzeitig wachsen die Kristalle in dem Hauptkristallisator zu einer Größe, die für die weitere Verarbeitung günstig ist.

Bevorzugt werden zur Gewinnung kristallinen Monosaccharides Kristalle des Monosaccharides mit einem mittleren Durchmesser von 200 bis 400 µm und/oder einer Reinheit von > 99 % abgetrennt.

Das abgetrennte kristalline Monosaccharid eignet sich aufgrund der Größe und der Reinheit gut zur weiteren Verarbeitung. Die Größe und die Reinheit des abgetrennten kristallinen Monosaccharides ergibt sich aus der erfindungsgemäßen Verfahrensführung.

In bestimmten Ausführungsformen wird das Prä-Kristallisationsmagma in den Vorkristallisatoren und/oder die Kristallsuspension in dem Hauptkristallisator um 0,1 bis 5,0 K/h abgekühlt.

Durch die Veränderung der Abkühlrate kann die Kristallwachstumsgeschwindigkeit beeinflusst werden. Bei einer Abkühlrate von 0,1 bis 5,0 K/h wird im metastabilen Bereich kristallisiert und die Bildung von Feinkorn durch unkontrollierte Primärkeimbildung oder Sekundärkeimbildung wird weitestgehend vermieden. Somit kann ein homogenes Kristallwachstum und eine geringe Partikelgrößenverteilung erreicht werden.

In bestimmten Ausführungsformen umfasst das Abtrennen von Kristallen des Monosaccharides aus der Kristallsuspension eine Zentrifugation von Kristallsuspension, wobei auch das Abtrennen kontinuierlich erfolgen kann, wenn die Zentrifugation alternierend in verschiedenen Zentrifugen erfolgt.

In bevorzugten Ausführungsformen umfasst das Verfahren einen Schritt, in welchem aus einer monosaccharidhaltigen Lösung durch Eindampfen die Lösung mit Monosaccharid, bevorzugt mit einer Übersättigung von 0 bis 60 %, und/oder die Lösung, die das Monosaccharid enthält und die bevorzugt übersättigt ist, gebildet wird.

Darüber hinaus kann das Verfahren einen Trocknungsschritt, beispielsweise in einem Wirbelschicht- oder Trommeltrockner, umfassen, an den sich bevorzugt eine Produktkühlung, wenn erforderlich mit konditionierter Luft, anschließt.

Teil der vorliegenden Erfindung ist auch ein kristallines Monosaccharid mit einem mittleren Durchmesser von 200 bis 400 µm und/oder einer Reinheit von >99 %.

Teil der vorliegenden Erfindung ist auch ein kristallines Monosaccharid, erhalten durch ein erfindungsgemäßes Verfahren oder unter Verwendung einer erfindungsgemäßen Vorrichtung.

Für Vorteile, Erklärungen und bevorzugte Ausführungsformen des kristallinen Monosaccharides wird auch auf die Ausführungen zu dem erfindungsgemäßen Verfahren und der Vorrichtung verwiesen, sofern sich aus der Beschreibung nichts Gegenteiliges ergibt.

Der Erfindung liegt auch die Aufgabe zu Grunde, eine Vorrichtung zur Gewinnung eines kristallinen Monosaccharides, insbesondere zur Durchführung des kontinuierlichen Verfahrens nach einem der Ansprüche 1 bis 14, anzugeben. Die erfindungsgemäße Vorrichtung umfasst einen Hauptkristallisator mit Mitteln zur kontinuierlichen Durchführung einer Verdampfungs- und/oder Kühlungskristallisation an einer Kristallsuspension zur Erzeugung eines Kristallwachstums von kristallinem Monosaccharid in der Kristallsuspension, Mittel zum Abtrennen von Kristallen des Monosaccharides aus der Kristallsuspension. Die erfindungsgemäße Vorrichtung umfasst ferner eine Kaskade zur kontinuierlichen Bildung einer Masse an Kristallisationsmagma für den Hauptkristallisator. Die Kaskade umfasst in Reihe geschaltet mindestens eine erste und eine letzte Stufe mit jeweils mindestens einem Vorkristallisator, Mittel zur Beimpfung einer Lösung mit Monosaccharid mit Monosaccharid-Saatkristallen in mindestens einem Vorkristallisator der ersten Stufe, um ein Prä-Kristallisationsmagma zu erhalten, und Mittel zur Durchführung einer Kühlungskristallisation und/oder Verdampfungskristallisation an dem Prä-Kristallisationsmagma in dem mindestens einem Vorkristallisator der ersten Stufe zur Bildung einer Masse an Kristallisationsmagma für die nachgeschaltete Stufe, und Mittel zum Zuführen einer Lösung mit Monosaccharid und einer Masse an Kristallisationsmagma aus der vorgeschalteten Stufe in den mindestens einen Vorkristallisator der letzten Stufe, um ein Prä-Kristallisationsmagma zu erhalten, und Mittel zur Durchführung einer Kühlungskristallisation und/oder Verdampfungskristallisation an dem Prä-Kristallisationsmagma in dem mindestens einem Vorkristallisator der letzten Stufe zur Bildung einer Masse an Kristallisationsmagma für den Hauptkristallisator. Die erfindungsgemäße Vorrichtung umfasst ferner Mittel zur kontinuierlichen Zuführung einer Lösung, die das Monosaccharid enthält, und einer Masse eines Kristallisationsmagmas aus dem mindestens einem Vorkristallisator der letzten Stufe der Kaskade in den Hauptkristallisator zur Bildung der Kristallsuspension.

Für Vorteile, Erklärungen und bevorzugte Ausführungsformen wird auch auf die Ausführungen zu dem erfindungsgemäßen Verfahren verwiesen, die sich auch auf die Vorrichtung beziehen sofern sich aus der Beschreibung nichts Gegenteiliges ergibt. Darüber hinaus werden bestimmte bevorzugte Ausführungen in den Unteransprüchen angegeben:
In einer bevorzugten Ausführungsform umfasst jede Stufe einen einzigen Vorkristallisator und die Kaskade Mittel zur kontinuierlichen Bildung einer Masse an Kristallisationsmagma aus dem Prä-Kristallisationsmagma in den Vorkristallisatoren mittels Verdampfungskristallisation.

In einer weiteren bevorzugten Ausführungsform umfasst jede Stufe zwei bis drei Vorkristallisatoren und die Kaskade Mittel zur diskontinuierlichen Bildung einer Masse von Kristallisationsmagma mittels Kühlungskristallisation und/oder zur kontinuierlichen Bildung einer Masse von Kristallisationsmagma mittels Verdampfungskristallisation in den Vorkristallisatoren jeder Stufe aus dem Prä-Kristallisationsmagma. Ferner umfasst die Kaskade bei diskontinuierlicher Bildung einer Masse an Kristallisationsmagma mittels Kühlungskristallisation Mittel zur kontinuierlichen Zuführung einer Masse an Kristallisationsmagma, alternierend aus den Vorkristallisatoren der letzten Stufe, in den Hauptkristallisator.

In bestimmten Ausführungsformen umfasst mindestens eine Stufe der Kaskade mehr als einen Vorkristallisator und die Vorkristallisatoren derselben Stufe weisen jeweils Mittel zur Bildung vorzugsweise gleicher Massen an Kristallisationsmagma auf.

Zu beachten ist dabei, dass nicht jede Stufe der Kaskade die gleiche Anzahl an Vorkristallisatoren aufweisen muss. Die Vorkristallisatoren können in vielfältiger Weise je Stufe untereinander verbunden sein.

Bevorzugt ist in bestimmten Ausführungsformen, dass die erste Stufe ein bis zwei, bevorzugt zwei, Vorkristallisatoren, die letzte Stufe zwei bis vier, bevorzugt drei, Vorkristallisatoren und eine weitere Stufe zwei bis vier, bevorzugt zwei, Vorkristallisatoren aufweist und die Kaskade Mittel aufweist, um in den Vorkristallisatoren jeder Stufe aus dem Prä-Kristallisationsmagma diskontinuierlich mittels Kühlungskristallisation eine Masse an Kristallisationsmagma zu bilden und die Kaskade Mittel aufweist, um dem Hauptkristallisator die Masse an Kristallisationsmagma kontinuierlich und alternierend aus den Vorkristallisatoren der letzten Stufe zuzuführen.

In bestimmten Ausführungsformen sind die Stufen bzw. der mindestens eine Vorkristallisator jeder Stufe derart miteinander verbunden, dass es möglich ist, einzelne weitere Stufen auszulassen.

Dies ist insbesondere vorteilhaft, um die Anlage kontinuierlich warten oder reinigen zu können. Ferner ist dies vorteilhaft, um geringere Mengen an Kristallisationsmagma zu erzeugen, falls für die Hauptkristallisation weniger Kristallisationsmagma benötigt wird.

In bestimmten Ausführungsformen umfasst die Vorrichtung mindestens eine Zentrifuge zum Abtrennen von Kristallen des Monosaccharides aus der Kristallsuspension. In bestimmten Ausführungsformen umfasst die Vorrichtung zum kontinuierlichen Abtrennen mehrere Zentrifugen, wobei die Zentrifugation in den mehreren Zentrifugen vorzugsweise jeweils batchweise erfolgt.

In bestimmten Ausführungsformen umfasst die Vorrichtung eine Trocknungseinheit, insbesondere einen Wirbelschicht- oder Trommeltrockner, an die sich bevorzugt eine Produktkühlung, wenn erforderlich mit konditionierter Luft, anschließt.

In bevorzugten Ausführungsformen umfasst die Vorrichtung eine Verdampfstation, in der aus einer monosaccharidhaltigen Lösung durch Eindampfen und bevorzugt Einstellung einer geeigneten Verdampfungsrate die Lösung mit Monosaccharid, bevorzugt mit einer Übersättigung von 0 bis 60 %, und/oder die Lösung, die das Monosaccharid enthält und die bevorzugt übersättigt ist, gebildet wird.

Bevorzugt sind die Vorkristallisatoren derart ausgebildet, dass die Masse an Kristallisationsmagma, die in den Vorkristallisatoren gebildet wird, ausgehend von der ersten Stufe mit jeder Stufe um den Faktor 2 bis 12, bevorzugt 4 bis 7, steigt.

Bevorzugt umfasst die Kaskade zwischen der ersten und der letzten Stufe eine bis acht, bevorzugt eine bis drei, am bevorzugtesten eine, in Reihe geschaltete weitere Stufe bzw. Stufen, wobei die weitere Stufe bzw. weiteren Stufen jeweils mindestens einen Vorkristallisator aufweist bzw. aufweisen. Ferner weist die Kaskade bevorzugt Mittel auf, um in den mindestens einen Vorkristallisator jeder weiteren Stufe Lösung mit Monosaccharid und eine Masse an Kristallisationsmagma aus der vorgeschalteten Stufe zu führen, um Prä-Kristallisationsmagma zu erhalten, und Mittel zur Bildung einer Masse an Kristallisationsmagma in dem mindestens einem Vorkristallisator jeder weiteren Stufe für die nachgeschaltete Stufe aus dem Prä-Kristallisationsmagma diskontinuierlich mittels Kühlungskristallisation und/oder kontinuierlich mittels Verdampfungskristallisation.

Bevorzugt umfasst die Vorrichtung Mittel, um Monosaccharid-Saatkristalle mit einem mittleren Durchmesser von 5 bis 30 µm, bevorzugt von 10 bis 20 µm, bereitzustellen.

In bevorzugten Ausführungsform weist der Hauptkristallisator Mittel auf, um einen Temperaturgradienten der Kristallsuspension über die Länge des Hauptkristallisators von 70 bis 15 °C und bevorzugt von 45 bis 25 °C einzustellen.

In bestimmten Ausführungsformen weist der Hauptkristallisator Mittel auf, um in dem Hauptkristallisator an einer Kristallsuspension kontinuierlich eine Kühlungskristallisation durchzuführen und die Kristallsuspension in dem Hauptkristallisator von bevorzugt 70 - 30 °C bevorzugt bis auf 35 - 15 °C abzukühlen.

In bestimmten Ausführungsformen weist der Hauptkristallisator Mittel auf, um in dem Hauptkristallisator an einer Kristallsuspension kontinuierlich eine Kühlungskristallisation durchzuführen und die Kristallsuspension in dem Hauptkristallisator von bevorzugt 70 - 33 °C bevorzugt bis auf 32 - 15 °C abzukühlen.

In einer bevorzugten Ausführungsform handelt es sich bei der Vorrichtung um eine Vorrichtung zur Gewinnung kristalliner Allulose und der Hauptkristallisator weist Mittel auf, um in dem Hauptkristallisator an einer Kristallsuspension kontinuierlich eine Kühlungskristallisation durchzuführen und um die Kristallsuspension in dem Hauptkristallisator von bevorzugt 45 - 35 °C bevorzugt bis auf 30 - 20 °C abzukühlen.

Bevorzugt weisen die Vorkristallisatoren jeweils einen Rührer mit einem spezifischen Leistungseintrag von 0,1 bis 4 kW/m³, bevorzugt von 0,5 bis 2,0 kW/m³, auf.

Denkbar ist auch, dass jeder Vorkristallisator mehrere Rührer aufweist, um denselben oben beschriebenen technischen Effekt zu erreichen.

In bevorzugten Ausführungsformen sind die Mittel zur kontinuierlichen Zuführung einer Lösung, die das Monosaccharid enthält, und zur kontinuierlichen Zuführung einer Masse an Kristallisationsmagma in den Hauptkristallisator derart gestaltet, dass dem Hauptkristallisator die Lösung, die das Monosaccharid enthält, und eine Masse an Kristallisationsmagma in einem Massenverhältnis von 1:5 bis 1:20, bevorzugt 1:7 bis 1:11, zugeführt wird.

In bestimmten Ausführungsformen umfasst die Vorrichtung Mittel, um das Prä-Kristallisationsmagma in den Vorkristallisatoren und/oder die Kristallsuspension in dem Hauptkristallisator um 0,1 bis 5,0 K/h abzukühlen.

Bei dem Monosaccharid des erfindungsgemäßen Verfahrens oder der erfindungsgemäßen Vorrichtung oder dem erfindungsgemäßen Monosaccharid handelt es sich insbesondere um ein Monosaccharid mit einem Schmelzpunkt von 90 bis 165 °C. Insbesondere handelt es sich um ein Monosaccharid der D-Konfiguration. Besonders bevorzugt handelt es sich um eine Hexulose, eine Hexose, eine Pentose oder eine Tetrose mit einem Schmelzpunkt von 90 bis 165 °C. Ganz besonders bevorzugt handelt es sich bei dem Monosaccharid um eine Hexulose, insbesondere Psicose (Allulose), insbesondere D-Psicose.

Mit Bezug auf die Figuren wird die Erfindung an einem Ausführungsbeispiel erläutert.

Dabei zeigen
- Fig. 1: einen Hauptkristallisator nach einer Vorrichtung der vorliegenden Erfindung und in einem Verfahren der vorliegenden Erfindung;
- Fig. 2: Hauptkristallisator und Vorkristallisatoren in einer Kaskade mit drei Stufen mit jeweils einem Vorkristallisator nach einer Vorrichtung der vorliegenden Erfindung und in einem Verfahren der vorliegenden Erfindung;
- Fig. 3: Hauptkristallisator und Vorkristallisatoren in einer Kaskade mit drei Stufen mit jeweils mehreren Vorkristallisatoren nach einer Vorrichtung der vorliegenden Erfindung und in einem Verfahren der vorliegenden Erfindung.

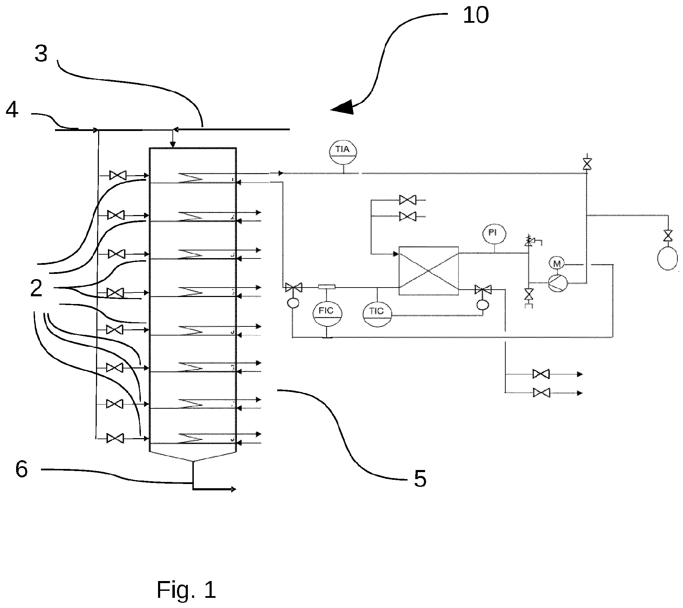

Fig. 1 zeigt einen Hauptkristallisator 10 in einer erfindungsgemäßen Vorrichtung zur Durchführung des erfindungsgemäßen Verfahrens. In diesem Beispiel sind die Lösung, die das Monosaccharid enthält, und die Lösung mit Monosaccharid identisch, d.h. sie enthalten dieselben Bestandteile in gleichen Mengen. Die Lösung wird in einer Verdampfstation eingedickt.

Der Hauptkristallisator 10 weist Injektionspunkte 2 für eine Lösung, die das Monosaccharid enthält, auf. Die Injektionspunkte 2 sind entlang der Höhe des Hauptkristallisators 10 und über den Umfang des Hauptkristallisators 10 verteilt. In diesem Beispiel bilden vier Injektionspunkte auf einer Höhe einen Injektionsring. Über die Höhe des Hauptkristallisators 10 sind acht solcher Injektionsringe verteilt. Die Ventile sind so getaktet, dass alle Injektionspunkte eines Injektionsringes geöffnet oder geschlossen sind.

Eine Masse an Kristallisationsmagma wird aus einer Leitung 3 von der letzten Stufe der Kaskade wird gemeinsam mit Lösung, die das Monosaccharid enthält, aus einer Leitung 4 oben in den Hauptkristallisator 10 eingeleitet.

Der Hauptkristallisator 10 weist in diesem Beispiel innen acht separate Wärmetauscher 5 zur Einstellung eines Temperaturprofils auf. Die Wärmetauscher 5 sind über die Höhe des Hauptkristallisators 10 verteilt und werden über je einen Wasserkreislauf zum Erwärmen/Kühlen der Kristallsuspension versorgt. Der Durchfluss und die Temperatur des Kreislaufwassers werden geregelt, so dass auf die Produkttemperatur/das Temperaturprofil kontrolliert Einfluss genommen werden kann.

Von dem Hauptkristallisator 10 führt eine Ableitung 6 zu einer Zentrifugenstation, in welcher Kristalle des Monosaccharides aus der Kristallsuspension abgetrennt werden.

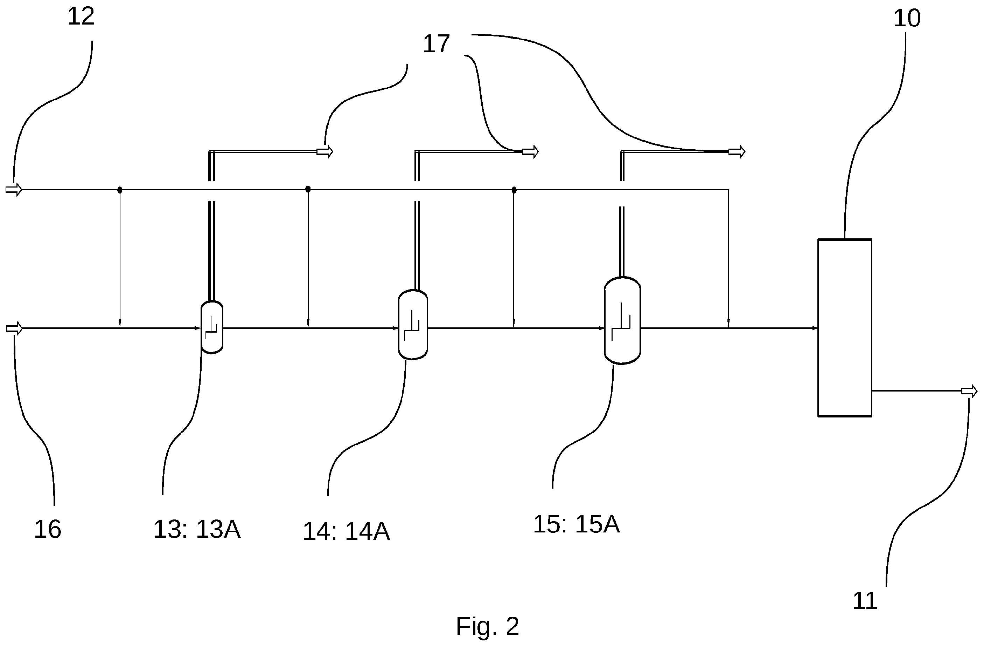

Fig. 2 zeigt eine Ausführungsform der Erfindung. In einem Hauptkristallisator 10 wird an einer Kristallsuspension kontinuierlich eine Kühlungskristallisation durchgeführt, um in der Kristallsuspension kristallines Monosaccharid aufwachsen zu lassen. Der Hauptkristallisator 10 ist ein vertikaler Kühlungskristallisator mit oszillierenden Kühlrohrbündeln. Aus dem Hauptkristallisator 10 wird kontinuierlich Kristallsuspension abgeführt und in einer Zentrifugenstation 11 werden gewachsene Kristalle des Monosaccharides aus der Kristallsuspension abgetrennt, um kristallines Monosaccharid zu gewinnen. Dem Hauptkristallisator 10 wird kontinuierlich eine Lösung, die das Monosaccharid enthält, und eine Masse eines Kristallisationsmagmas zugeführt, um die Kristallsuspension bereitzustellen. Das Kristallisationsmagma stammt aus einer Kaskade zur kontinuierlichen Bildung einer Masse an Kristallisationsmagma.

In diesem Beispiel sind die Lösung, die das Monosaccharid enthält, und die Lösung mit Monosaccharid identisch, d.h. sie enthalten dieselben Bestandteile in gleichen Mengen. In diesem Beispiel wird in einer Verdampfstation 12 eine Lösung, die das Monosaccharid enthält, bzw. die Lösung mit Monosaccharid mit einem Trockensubstanzkonzentration von 82 % und mit einer Temperatur von 40 °C bereitgestellt. Diese Lösung, die das Monosaccharid umfasst, wird so den Vorkristallisatoren 13A, 14A, 15A und dem Hauptkristallisator 10 zugeführt.

Die Kaskade umfasst drei in Reihe geschaltete Stufen 13, 14, 15 mit jeweils einem Vorkristallisator 13A, 14A, 15A. In jedem Vorkristallisator 13A, 14A, 15A wird kontinuierlich eine Verdampfungskristallisation durchgeführt. In den Vorkristallisator 15A der letzten Stufe 15 wird Lösung mit Monosaccharid und eine Masse an Kristallisationsmagma aus der vorgeschalteten Stufe 14 zugeführt, um ein Prä-Kristallisationsmagma zu erhalten. In dem Vorkristallisator 15A der letzten Stufe 15 wird dann aus dem Prä-Kristallisationsmagma mittels Verdampfungskristallisation eine Masse an Kristallisationsmagma für den Hauptkristallisator 10 gebildet.

In dem Vorkristallisator 13A der ersten Stufe 13 wird Lösung mit Monosaccharid mit einer Saatsuspension (die Slurry) 16 mit Monosaccharid-Saatkristallen mit einem mittleren Kristalldurchmesser von 13 µm beimpft, um ein Prä-Kristallisationsmagma zu erhalten. Die Saatsuspension (die Slurry) mit Monosaccharid-Saatkristallen weist einen Kristallgehalt von 20 Gew.-% und eine Temperatur von 20 °C auf und wird mit einer Rate von 0,30 L/h bzw. 0,43 kg/h zugeführt. Lösung mit Monosaccharid wird dem Vorkristallisator 13A in einer Rate von 2,7 L/h zugeführt. Das Gemisch ergibt ein Prä-Kristallisationsmagma mit einem Kristallgehalt von 2,1 Gew.-%. Aus dem Prä-Kristallisationsmagma wird mittels Verdampfungskristallisation eine Masse an Kristallisationsmagma für den Vorkristallisator 14A der nachgeschalteten, mittleren Stufe 14 gebildet. Das Nettovolumen des Vorkristallisators 13A der ersten Stufe 13 beträgt 0,15 m³, Brüden 17 werden bei einer Temperatur von 63 °C mit einer Rate von 0,2 kg/h entlassen. Die Verweilzeit in dem Vorkristallisator 13A beträgt 43,3 h. Eine Masse an Kristallisationsmagma wird dem Vorkristallisator 14A der mittleren Stufe 14 mit einer Rate von 2,7 L/h, einer Temperatur von 63 °C, einem mittleren Kristalldurchmesser von 30 µm und einem Kristallgehalt von 27 Gew.-% zugeführt. Diesem Vorkristallisator 14A wird auch Lösung mit Monosaccharid mit einer Rate von 21,4 L/h zugeführt.

Das Gemisch ergibt ein Prä-Kristallisationsmagma mit einem Kristallgehalt von 3,2 Gew.-% und einer Temperatur von 42,7 °C. Aus dem Prä-Kristallisationsmagma wird mittels Verdampfungskristallisation eine Masse an Kristallisationsmagma für den Vorkristallisator 15A der nachgeschalteten, letzten Stufe 15 gebildet. Das Nettovolumen des Vorkristallisators 14A der mittleren Stufe 14 beträgt 1,0 m³, Brüden 17 werden bei einer Temperatur von 65 °C mit einer Rate von 1,8 kg/h entlassen. Die Verweilzeit beträgt 40,0 h und eine Masse an Kristallisationsmagma von 21,8 L/h mit einer Temperatur von 65 °C, einem mittleren Kristalldurchmesser von 60 µm und einem Kristallgehalt von 27 Gew.-% wird dem Vorkristallisator 15A der letzten Stufe 15 zugeführt. Dem Vorkristallisator 15A wird auch Lösung mit Monosaccharid mit einer Rate von 208 L/h zugeführt.

Das Gemisch ergibt ein Prä-Kristallisationsmagma mit einem Kristallgehalt von 2,7 Gew.-% und einer Temperatur von 42,5 °C. Aus dem Prä-Kristallisationsmagma wird mittels Verdampfungskristallisation eine Masse an Kristallisationsmagma für den Hauptkristallisator 10 gebildet. Das Nettovolumen des Vorkristallisators 15A der letzten Stufe 15 beträgt 6,0 m³, Brüden 17 werden bei einer Temperatur von 70 °C mit einer Rate von 14 kg/h entlassen. Die Verweilzeit beträgt 26,7 h und eine Masse an Kristallisationsmagma von 209 L/h mit einer Temperatur von 70 °C, einem mittleren Kristalldurchmesser von 120 µm und einem Kristallgehalt von 22,5 Gew.-% wird dem Hauptkristallisator 10 zugeführt. Dem Hauptkristallisator 10 wird auch Lösung mit Monosaccharid, die hier identisch mit der Lösung, die das Monosaccharid enthält, ist, in einer Rate von 1990 L/h zugeführt.

Das Gemisch ergibt eine Kristallsuspension mit einem Kristallgehalt von 2,2 Gew.-% und einer Temperatur von 43,0 °C. In der Kristallsuspension wird mittels Kühlungskristallisation kristallines Monosaccharid gebildet, vor allem aber wachsen Kristalle kristallinem Monosaccharides. Das Nettovolumen des Hauptkristallisators 10 beträgt 157,0 m³. Die Verweilzeit beträgt 73,0 h. Während dieser Zeit wird die Kristallsuspension mit 0,3 K/h abgekühlt. Kristallsuspension mit gebildetem kristallinem Monosaccharid wird mit einer Rate von 2100 L/h mit einer Temperatur von 19 °C, einem mittleren Kristalldurchmesser von 300 µm und einem Kristallgehalt von 35,3 Gew.-% einer Zentrifugenstation 11 zugeführt. Dort werden Kristalle des Monosaccharides durch Zentrifugation abgetrennt und so kristallines Monosaccharid gewonnen.

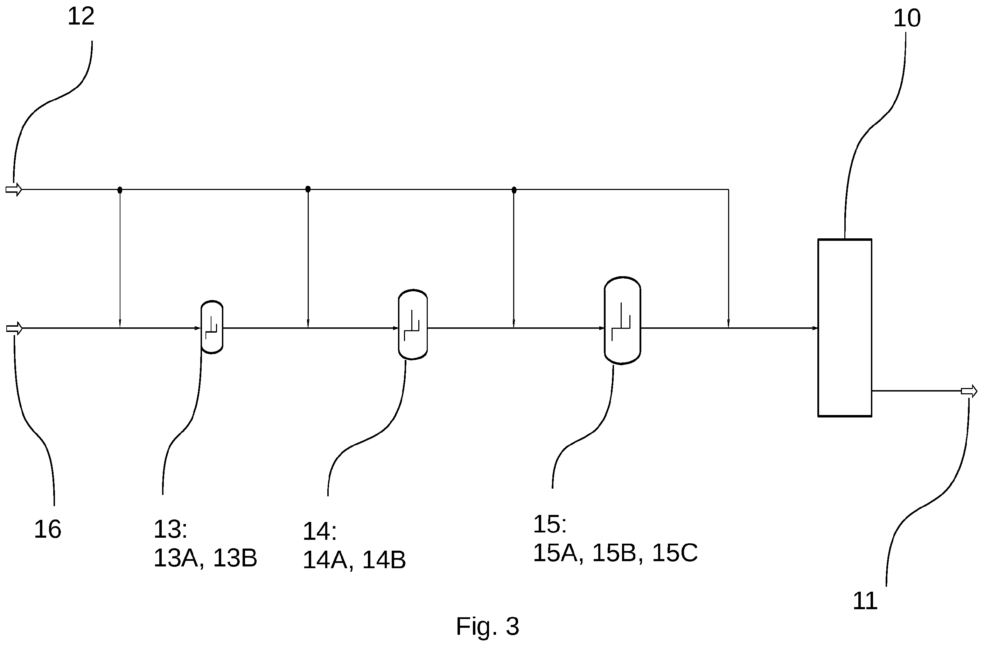

Fig. 3 zeigt eine weitere Ausführungsform der Erfindung. In einem Hauptkristallisator 10 wird an einer Kristallsuspension kontinuierlich eine Kühlungskristallisation durchgeführt, um in der Kristallsuspension Monosaccharidkristalle aufwachsen zu lassen. Der Hauptkristallisator 10 ist ein vertikaler Kühlungskristallisator mit oszillierenden Kühlrohrbündeln. Aus dem Hauptkristallisator 10 wird kontinuierlich Kristallsuspension abgeführt und in einer Zentrifugenstation 11 werden (gewachsene) Kristalle des Monosaccharides aus der Kristallsuspension abgetrennt, um kristallines Monosaccharid zu gewinnen. Dem Hauptkristallisator 10 wird kontinuierlich eine Lösung, die das Monosaccharid enthält, und eine Masse eines Kristallisationsmagmas zugeführt, um die Kristallsuspension bereitzustellen. Das Kristallisationsmagma stammt aus einer Kaskade zur kontinuierlichen Bildung einer Masse an Kristallisationsmagma.

In diesem Beispiel sind die Lösung, die das Monosaccharid enthält, und die Lösung mit Monosaccharid identisch, d.h. sie enthalten dieselben Bestandteile in gleichen Mengen. In diesem Beispiel wird in einer Verdampfstation 12 eine Lösung, die das Monosaccharid enthält, bzw. die Lösung mit Monosaccharid mit einer Trockensubstanzkonzentration von 82 % und mit einer Temperatur von 41 °C bereitgestellt. Diese Lösung, die das Monosaccharid umfasst, wird so den Vorkristallisatoren 13A, 13B, 14A, 14B, 15A, 15B und 15C und dem Hauptkristallisator 10 zugeführt.

Die Kaskade umfasst drei in Reihe geschaltete Stufen 13, 14, 15, wobei die erste Stufe 13 zwei Vorkristallisatoren 13A, 13B, die mittlere Stufe 14 zwei Vorkristallisatoren 14A, 14B und die letzte Stufe 15 drei Vorkristallisatoren 15A, 15B, 15C aufweist. In jedem Vorkristallisator 13A, 13B, 14A, 14B, 15A, 15B und 15C wird diskontinuierlich eine Kühlungskristallisation durchgeführt. In den Vorkristallisatoren 15A, 15B und 15C der letzten Stufe 15 wird Lösung mit Monosaccharid und eine Masse an Kristallisationsmagma aus der vorgeschalteten Stufe 14 zugeführt, um ein Prä-Kristallisationsmagma zu erhalten. In den Vorkristallisatoren 15A, 15B und 15C der letzten Stufe 15 wird dann aus dem Prä-Kristallisationsmagma mittels Kühlungskristallisation eine Masse an Kristallisationsmagma für den Hauptkristallisator 10 gebildet. Die Kühlungskristallisation in den drei Vorkristallisatoren 15A, 15B und 15C der letzten Stufe 15 verläuft jeweils diskontinuierlich. Die Kühlungskristallisation in den drei Vorkristallisatoren 15A, 15B und 15C ist aber so geschaltet, dass immer aus einem Vorkristallisator Kristallisationsmagma in den Hauptkristallisator 10 geführt werden kann, so dass eine kontinuierliche Zuführung an Kristallisationsmagma in den Hauptkristallisator 10 gewährleistet wird. Gleichzeitig können die anderen Vorkistallisatoren gereinigt bzw. befüllt werden.

In den zwei Vorkristallisatoren 13A, 13B der ersten Stufe 13 wird Lösung mit Monosaccharid mit einer Saatsuspension (einer Slurry) 16 mit Monosaccharid-Saatkristallen mit einem mittleren Kristalldurchmesser von 13 µm beimpft, um ein Prä-Kristallisationsmagma zu erhalten. Die Saatsuspension (die Slurry) 16 mit Monosaccharid-Saatkristallen weist einen Kristallgehalt von 20 Gew.-% und eine Temperatur von 20 °C auf und wird den Vorkristallisatoren 13A, 13B mit einer Rate von insgesamt 0,30 L/h bzw. 0,43 kg/h zugeführt. Die Lösung mit Monosaccharid wird den Vorkristallisatoren 13A, 13B mit einer Rate von insgesamt 2,6 L/h zugeführt. Das Gemisch ergibt ein Prä-Kristallisationsmagma mit einem Kristallgehalt von 2,2 Gew.-%. Aus dem Prä-Kristallisationsmagma wird mittels Kühlungskristallisation eine Masse an Kristallisationsmagma für die zwei Vorkristallisatoren 14A, 14B der nachgeschalteten, mittleren Stufe 14 gebildet. Das Nettovolumen der Vorkristallisatoren 13A, 13B der ersten Stufe 13 beträgt jeweils 0,070 m³. Die Verweilzeit in den Vorkristallisatoren 13A, 13B beträgt 43,3 h, die Kühlungsrate 0,3 K/h. Eine Masse an Kristallisationsmagma wird den Vorkristallisatoren 14A, 14B der mittleren Stufe 14 in einer Rate von insgesamt 2,7 L/h, mit einer Temperatur von 27 °C, einem mittleren Kristalldurchmesser von 30 µm und einem Kristallgehalt von 27 Gew.-% zugeführt. Diesen Vorkristallisatoren 14A, 14B wird auch Lösung mit Monosaccharid in einer Rate von insgesamt 20,1 L/h zugeführt.

Das Gemisch ergibt ein Prä-Kristallisationsmagma mit einem Kristallgehalt von 3,4 Gew.-% und einer Temperatur von 40,0 °C. Aus dem Prä-Kristallisationsmagma wird mittels Kühlungskristallisation eine Masse an Kristallisationsmagma für die drei Vorkristallisatoren 15A, 15B, 15C der nachgeschalteten, letzten Stufe 15 gebildet. Das Nettovolumen der Vorkristallisatoren 14A, 14B der mittleren Stufe 14 beträgt jeweils 0,50 m³. Die Verweilzeit in den Vorkristallisatoren 14A, 14B der mittleren Stufe 14 beträgt 40,0 h, die Kühlungsrate 0,3 K/h. Eine Masse an Kristallisationsmagma von insgesamt 21,8 L/h mit einer Temperatur von 28 °C, einem mittleren Kristalldurchmesser von 60 µm und einem Kristallgehalt von 27 Gew.-% wird den Vorkristallisatoren 15A, 15B, 15C der letzten Stufe 15 zugeführt. Den Vorkristallisatoren 15A, 15B, 15C der letzten Stufe 15 wird auch Lösung mit Monosaccharid in einer Rate von insgesamt 197 L/h zugeführt.

Das Gemisch ergibt ein Prä-Kristallisationsmagma mit einem Kristallgehalt von 2,8 Gew.-% und einer Temperatur von 40,0 °C. Aus dem Prä-Kristallisationsmagma wird mittels Kühlungskristallisation eine Masse an Kristallisationsmagma für den Hauptkristallisator 10 gebildet. Das Nettovolumen der Vorkristallisatoren 15A, 15B, 15C der letzten Stufe 15 beträgt jeweils 2,2 m³. Die Verweilzeit in den Vorkristallisatoren 15A, 15B, 15C der letzten Stufe beträgt 26,7 h, die Kühlungsrate 0,3 K/h. Eine Masse an Kristallisationsmagma in einer Rate von 209 L/h, mit einer Temperatur von 32 °C, einem mittleren Kristalldurchmesser von 120 µm und einem Kristallgehalt von 22,5 Gew.-% wird dem Hauptkristallisator 10 zugeführt. Dem Hauptkristallisator 10 wird auch Lösung mit Monosaccharid, die hier identisch mit der Lösung, die das Monosaccharid enthält, ist, in einer Rate von 1990 L/h zugeführt.

Das Gemisch ergibt eine Kristallsuspension mit einem Kristallgehalt von 2,2 Gew.-% und einer Temperatur von 40,0 °C. In der Kristallsuspension wird mittels Kühlungskristallisation kristallines Monosaccharid gebildet. Das Nettovolumen des Hauptkristallisators 10 beträgt 157,0 m³. Die Verweilzeit beträgt 73,0 h. Während dieser Zeit wird die Kristallsuspension mit 0,3 K/h abgekühlt. Kristallsuspension mit gebildetem kristallinem Monosaccharid wird mit einer Rate von 2100 L/h, mit einer Temperatur von 19 °C, einem mittleren Kristalldurchmesser von 300 µm und einem Kristallgehalt von 35,0 Gew.-% einer Zentrifugenstation 11 zugeführt. Dort wird kristallines Monosaccharid durch Zentrifugation abgetrennt und gewonnen.

Die Reinheit der Kristalle beträgt in den Beispielen > 99 %. Die Dichte der Lösung mit Monosaccharid beträgt ca. 1,36 kg/L. Die Dichte des Kristallisationsmagmas beträgt ca. 1,44 kg/L. Jeder Vorkristallisator in den Beispielen weist einen Rührer mit einem spezifischen Leistungseintrag von 0,5 bis 2,0 kW/m³ auf.

### Bezugszeichenliste

- 2: Injektionspunkte
- 3: Leitung für eine Masse an Kristallisationsmagma
- 4: Leitung für eine Lösung, die das Monosaccharid enthält
- 5: Wärmetauscher
- 6: Ableitung zu einer Zentrifugenstation
- 10: Hauptkristallisator
- 11: Zentrifugenstation
- 12: Verdampfstation
- 13: erste Stufe der Kaskade
- 13A, 13B: Vorkristallisator(en) der ersten Stufe
- 14: zweite/weitere Stufe der Kaskade
- 14A, 14B: Vorkristallisator(en) der zweiten Stufe
- 15: letzte Stufe der Kaskade
- 15A, 15B, 15C: Vorkristallisator(en) der letzten Stufe
- 16: Saatsuspension (Slurry)
- 17: Brüden

## Patentansprüche

1. Kontinuierliches Verfahren zur Gewinnung eines kristallinen Monosaccharides, umfassend:
- eine kontinuierliche Kristallisation des Monosaccharides in einem Hauptkristallisator (10),
- wobei in dem Hauptkristallisator (10) an einer Kristallsuspension kontinuierlich eine Verdampfungs- und/oder Kühlungskristallisation durchgeführt wird, um in der Kristallsuspension Kristalle des Monosaccharides wachsen zu lassen,
- ein Abtrennen von Kristallen des Monosaccharides aus der Kristallsuspension, um kristallines Monosaccharid zu gewinnen;
- die kontinuierliche Bildung einer Masse an Kristallisationsmagma für den Hauptkristallisator (10) in einer Kaskade,
- wobei die Kaskade in Reihe geschaltet mindestens eine erste (13) und eine letzte Stufe (15) umfasst und jede Stufe mindestens einen Vorkristallisator (13A, 15A) umfasst,
- wobei in dem mindestens einen Vorkristallisator (13A) der ersten Stufe (13) Lösung mit Monosaccharid durch Monosaccharid-Saatkristalle beimpft wird, um ein Prä-Kristallisationsmagma zu erhalten, und aus dem Prä-Kristallisationsmagma mittels Kühlungskristallisation und/oder Verdampfungskristallisation eine Masse an Kristallisationsmagma für die nachgeschaltete Stufe (14, 15) gebildet wird, und
- wobei in den mindestens einen Vorkristallisator (15A, 15B, 15C) der letzten Stufe (15) Lösung mit Monosaccharid und eine Masse an Kristallisationsmagma aus der vorgeschalteten Stufe zugeführt wird, um ein Prä-Kristallisationsmagma zu erhalten, und in dem mindestens einem Vorkristallisator (15A, 15B, 15C) der letzten Stufe (15) aus dem Prä-Kristallisationsmagma mittels Kühlungskristallisation und/oder Verdampfungskristallisation eine Masse an Kristallisationsmagma für den Hauptkristallisator (10) gebildet wird;
- das kontinuierliche Zuführen einer Lösung, die das Monosaccharid enthält, und einer Masse eines Kristallisationsmagmas aus dem mindestens einem Vorkristallisator (15A, 15B, 15C) der letzten Stufe (15) der Kaskade in den Hauptkristallisator (10), um die Kristallsuspension bereitzustellen.

2. Verfahren nach Anspruch 1, wobei jede Stufe (13, 14, 15) der Kaskade einen einzigen Vorkristallisator (13A, 14A, 15A) umfasst und in dem Vorkristallisator (13A, 14A, 15A) jeder Stufe (13, 14, 15) aus dem Prä-Kristallisationsmagma mittels Verdampfungskristallisation kontinuierlich eine Masse an Kristallisationsmagma gebildet wird.

3. Verfahren nach Anspruch 1, wobei jede Stufe (13, 14, 15) zwei bis drei Vorkristallisatoren (13A, 13B, 14A, 14B, 15A, 15B, 15C) umfasst und in den Vorkristallisatoren (13A, 13B, 14A, 14B, 15A, 15B, 15C) jeder Stufe (13, 14, 15) aus dem Prä-Kristallisationsmagma diskontinuierlich mittels Kühlungskristallisation und/oder kontinuierlich mittels Verdampfungskristallisation eine Masse an Kristallisationsmagma gebildet wird, wobei die Masse an Kristallisationsmagma, die dem Hauptkristallisator (10) kontinuierlich zugeführt wird, bei diskontinuierlicher Bildung mittels Kühlungskristallisation alternierend aus den Vorkristallisatoren (15A, 15B, 15C) der letzten Stufe (15) zugeführt wird.

4. Verfahren nach einem der vorherigen Ansprüche, insbesondere Anspruch 1 oder 3, wobei mindestens eine Stufe (13, 14, 15) mehr als einen Vorkristallisator umfasst und die Vorkristallisatoren (13A, 13B, 14A, 14B, 15A, 15B, 15C) derselben Stufe (13, 14, 15) jeweils vorzugsweise die gleiche Masse an Kristallisationsmagma bilden.

5. Verfahren nach einem der vorherigen Ansprüche, wobei die Kaskade zwischen der ersten (13) und der letzten (15) Stufe eine bis acht, bevorzugt eine bis drei, am bevorzugtesten eine, in Reihe geschaltete weitere Stufe (14) bzw. Stufen umfasst, wobei die weitere Stufe (14) bzw. weiteren Stufen jeweils mindestens einen Vorkristallisator (14A, 14B) aufweist bzw. aufweisen, in welchem bzw. welchen Lösung mit Monosaccharid und eine Masse an Kristallisationsmagma aus der vorgeschalteten Stufe (13) zugeführt wird, um Prä-Kristallisationsmagma zu erhalten, und wobei in dem mindestens einen Vorkristallisator (14A, 14B) jeder weiteren Stufe (14) aus dem Prä-Kristallisationsmagma diskontinuierlich mittels Kühlungskristallisation und/oder kontinuierlich mittels Verdampfungskristallisation eine Masse an Kristallisationsmagma für die nachgeschaltete Stufe (15) gebildet wird.

6. Verfahren nach einem der vorherigen Ansprüche, wobei die Monosaccharid-Saatkristalle einen mittleren Durchmesser von 5 bis 50 µm, bevorzugt 10 bis 20 µm, aufweisen.

7. Verfahren nach einem der vorherigen Ansprüche, wobei ein Temperaturgradient der Kristallsuspension über die Länge des Hauptkristallisators (10) von 70 bis 15 °C und bevorzugt von 45 bis 25 °C eingestellt wird und/oder die Verweildauer der Kristallsuspension in dem Hauptkristallisator (10) 30 bis 70 Stunden beträgt.

8. Verfahren nach einem der vorherigen Ansprüche, wobei der Inhalt jedes Vorkristallisators (13A, 13B, 14A, 14B, 15A, 15B, 15C), bevorzugt ein oder mehrere Lösungen, Suspensionen, Prä-Kristallisationsmagma und/oder Kristallisationsmagma, von einem Rührer mit einem spezifischen Leistungseintrag von 0,1 bis 4,0 kW/m³, bevorzugt von 0,5 bis 2,0 kW/m³, angetrieben wird.

9. Verfahren nach einem der vorherigen Ansprüche, wobei die Lösung mit Monosaccharid für die Vorkristallisatoren eine Übersättigung von 0 bis 60 % aufweist und/oder die Lösung, die das Monosaccharid enthält, bei Zuführung in den Hauptkristallisator (10) übersättigt ist.

10. Verfahren nach einem der vorherigen Ansprüche, wobei das Prä-Kristallisationsmagma in den Vorkristallisatoren (13A, 13B, 14A, 14B, 15A, 15B, 15C) und/oder die Kristallsuspension im Hauptkristallisator (10) um 0,1 bis 5,0 K/h abgekühlt wird.

11. Vorrichtung zur Gewinnung eines kristallinen Monosaccharides, insbesondere zur Durchführung des kontinuierlichen Verfahrens nach einem der Ansprüche 1 bis 10, umfassend
- einen Hauptkristallisator (10) mit
- Mitteln zur kontinuierlichen Durchführung einer Verdampfungs- und/oder Kühlungskristallisation an einer Kristallsuspension zur Erzeugung eines Kristallwachstums von kristallinem Monosaccharid in der Kristallsuspension,
- Mittel zum Abtrennen von Kristallen des Monosaccharides aus der Kristallsuspension,
- eine Kaskade zur kontinuierlichen Bildung einer Masse an Kristallisationsmagma für den Hauptkristallisator, wobei die Kaskade umfasst:
- in Reihe geschaltet mindestens eine erste (13) und eine letzte (15) Stufe mit jeweils mindestens einem Vorkristallisator (13A, 13B, 15A, 15B, 15C),
- Mittel zur Beimpfung einer Lösung mit Monosaccharid mit Monosaccharid-Saatkristallen in mindestens einem Vorkristallisator (13A, 13B) der ersten Stufe (13), um ein Prä-Kristallisationsmagma zu erhalten, und Mittel zur Durchführung einer Kühlungskristallisation und/oder Verdampfungskristallisation an dem Prä-Kristallisationsmagma in dem mindestens einem Vorkristallisator (13A, 13B) der ersten Stufe (13) zur Bildung einer Masse an Kristallisationsmagma für die nachgeschaltete Stufe, und
- Mittel zum Zuführen einer Lösung mit Monosaccharid und einer Masse an Kristallisationsmagma aus der vorgeschalteten Stufe in den mindestens einen Vorkristallisator (15A, 15B, 15C) der letzten Stufe (15), um ein Prä-Kristallisationsmagma zu erhalten, und Mittel zur Durchführung einer Kühlungskristallisation und/oder Verdampfungskristallisation an dem Prä-Kristallisationsmagma in dem mindestens einem Vorkristallisator (15A, 15B, 15C) der letzten Stufe zur Bildung einer Masse an Kristallisationsmagma für den Hauptkristallisator (10);
- Mittel zur kontinuierlichen Zuführung einer Lösung, die das Monosaccharid enthält, und einer Masse eines Kristallisationsmagmas aus dem mindestens einem Vorkristallisator (15A, 15B, 15C) der letzten Stufe (15) der Kaskade in den Hauptkristallisator (10) zur Bildung der Kristallsuspension.

12. Vorrichtung nach Anspruch 11, wobei jede Stufe (13, 14, 15) einen einzigen Vorkristallisator (13A, 14A, 15A) und die Kaskade Mittel zur kontinuierlichen Bildung einer Masse an Kristallisationsmagma aus dem Prä-Kristallisationsmagma in den Vorkristallisatoren (13A, 14A, 15A) mittels Verdampfungskristallisation umfasst.

13. Vorrichtung nach Anspruch 11, wobei jede Stufe (13, 14, 15) zwei bis drei Vorkristallisatoren (13A, 13B, 14A, 14B, 15A, 15B, 15C) und die Kaskade Mittel zur diskontinuierlichen Bildung einer Masse von Kristallisationsmagma mittels Kühlungskristallisation und/oder zur kontinuierlichen Bildung einer Masse von Kristallisationsmagma mittels Verdampfungskristallisation in den Vorkristallisatoren (13A, 13B, 14A, 14B, 15A, 15B, 15C) jeder Stufe (13, 14, 15) aus dem Prä-Kristallisationsmagma umfasst sowie bei diskontinuierlicher Bildung einer Masse an Kristallisationsmagma mittels Kühlungskristallisation Mittel zur kontinuierlichen Zuführung einer Masse an Kristallisationsmagma, alternierend aus den Vorkristallisatoren (15A, 15B, 15C) der letzten Stufe (15), in den Hauptkristallisator.

14. Vorrichtung nach einem der vorherigen Ansprüche, insbesondere Anspruch 11 oder 13, wobei mindestens eine Stufe (13, 14, 15) mehr als einen Vorkristallisator (13A, 13B, 14A, 14B, 15A, 15B, 15C) umfasst und die Vorkristallisatoren (13A, 13B, 14A, 14B, 15A, 15B, 15C) derselben Stufe jeweils Mittel zur Bildung vorzugsweise gleicher Massen an Kristallisationsmagma aufweisen.

15. Vorrichtung nach einem der vorherigen Ansprüche, wobei die Kaskade zwischen der ersten (13) und der letzten (15) Stufe eine bis acht, bevorzugt eine bis drei, am bevorzugtesten eine, in Reihe geschaltete weitere Stufe (14) bzw. Stufen umfasst, wobei die weitere Stufe (14) bzw. weiteren Stufen jeweils mindestens einen Vorkristallisator (14A, 14B) aufweist bzw. aufweisen, und die Kaskade Mittel aufweist, um in den mindestens einen Vorkristallisator (14A, 14B) jeder weiteren Stufe (14) Lösung mit Monosaccharid und eine Masse an Kristallisationsmagma aus der vorgeschalteten Stufe (13) zu führen, um Prä-Kristallisationsmagma zu erhalten, und die Kaskade Mittel zur Bildung einer Masse an Kristallisationsmagma in dem mindestens einen Vorkristallisator (14A, 14B) jeder weiteren Stufe für die nachgeschaltete Stufe aus dem Prä-Kristallisationsmagma diskontinuierlich mittels Kühlungskristallisation und/oder kontinuierlich mittels Verdampfungskristallisation aufweist.

16. Verfahren nach einem der Ansprüche 1 bis 10 oder Vorrichtung nach einem der Ansprüche 11 bis 15, wobei es sich bei dem Monosaccharid um ein Monosaccharid, insbesondere eine Hexulose, eine Hexose, eine Pentose oder eine Tetrose, mit einem Schmelzpunkt von 90 bis 165 °C handelt, wobei es sich bei dem Monosaccharid besonders bevorzugt um eine Hexulose, insbesondere Psicose (Allulose), insbesondere D-Psicose, handelt.

## Claims

1. Continuous method for obtaining a crystalline monosaccharide, comprising:
- continuously crystallizing the monosaccharide in a main crystallizer (10),
- wherein evaporation and/or cooling crystallization is carried out continuously on a crystal suspension in the main crystallizer (10) in order to cause crystals of the monosaccharide to grow in the crystal suspension,
- separating crystals of the monosaccharide from the crystal suspension to obtain crystalline monosaccharide;
- continuously forming a mass of crystallization magma for the main crystallizer (10) in a cascade,
- wherein the cascade comprises at least one first (13) and one last stage (15) connected in series and each stage comprises at least one pre-crystallizer (13A, 15A),
- wherein solution having monosaccharide is inoculated by monosaccharide crystals in the at least one pre-crystallizer (13A) of the first stage (13) to obtain a pre-crystallization magma, and a mass of crystallization magma for the downstream stage (14, 15) is formed from the pre-crystallization magma by means of cooling crystallization and/or evaporation crystallization, and
- wherein solution having monosaccharide and a mass of pre-crystallization magma from the upstream stage is supplied into the at least one pre-crystallizer (15A, 15B, 15C) of the last stage (15), and a mass of crystallization magma for the main crystallizer (10) is formed in the at least one pre-crystallizer (15A, 15B, 15C) of the last stage (15) from the pre-crystallization magma by means of cooling crystallization and/or evaporation crystallization;
- continuously supplying a solution that contains the monosaccharide and a mass of a crystallization magma from the at least one pre-crystallizer (15A, 15B, 15C) of the last stage (15) of the cascade into the main crystallizer (10) in order to provide the crystal suspension.

2. Method as claimed in claim 1, wherein each stage (13, 14, 15) of the cascade comprises a single pre-crystallizer (13A, 14A, 15A) and a mass of crystallization magma is formed in the pre-crystallizer (13A, 14A, 15A) of each stage (13, 14, 15) from the pre-crystallization magma continuously by means of evaporation crystallization.

3. Method as claimed in claim 1, wherein each stage (13, 14, 15) comprises two to three pre-crystallizers (13A, 13B, 14A, 14B, 15A, 15B, 15C) and a mass of crystallization magma is formed in the pre-crystallizers (13A, 13B, 14A, 14B, 15A, 15B, 15C) of each stage (13, 14, 15) from the pre-crystallization magma discontinuously by means of cooling crystallization and/or continuously by means of evaporation crystallization, wherein the mass of crystallization magma, which is continuously supplied to the main crystallizer (10), is alternately supplied from the pre-crystallizers (15A, 15B, 15C) of the last stage (15) in the case of discontinuous formation by means of cooling crystallization.

4. Method as claimed in any one of the preceding claims, in particular claim 1 or 3, wherein at least one stage (13, 14, 15) comprises more than one pre-crystallizer and the pre-crystallizers (13A, 13B, 14A, 14B, 15A, 15B, 15C) of the same stage (13, 14, 15) each preferably form the same mass of crystallization magma.

5. Method as claimed in any one of the preceding claims, wherein the cascade between the first (13) and the last (15) stage comprises one to eight, preferably one to three, most preferably one further stage (14) or stages connected in series, wherein the further stage (14) or further stages each have at least one pre-crystallizer (14A, 14B), into which solution having monosaccharide and a mass of crystallization magma from the upstream stage (13) is supplied to obtain pre-crystallization magma, and wherein a mass of crystallization magma for the downstream stage (15) is formed in the at least one pre-crystallizer (14A, 14B) of each further stage (14) from the pre-crystallization magma discontinuously by means of cooling crystallization and/or continuously by means of evaporation crystallization.

6. Method as claimed in any one of the preceding claims, wherein the monosaccharide seed crystals have an average diameter of 5 to 50 µm, preferably 10 to 20 µm.

7. Method as claimed in any one of the preceding claims, wherein a temperature gradient of the crystal suspension over the length of the main crystallizer (10) from 70 to 15°C and preferably from 45 to 25°C is set and/or the dwell time of the crystal suspension in the main crystallizer (10) is 30 to 70 hours.

8. Method as claimed in any one of the preceding claims, wherein the content of each pre-crystallizer (13A, 13B, 14A, 14B, 15A, 15B, 15C), preferably one or more solutions, suspensions, pre-crystallization magma, and/or crystallization magma, is driven by a stirrer using a specific power introduction of 0.1 to 4.0 kW/m³, preferably of 0.5 to 2.0 kW/m³.

9. Method as claimed in any one of the preceding claims, wherein the solution having monosaccharide for the pre-crystallizers has an oversaturation of 0 to 60% and/or the solution that contains the monosaccharide is oversaturated upon supply into the main crystallizer (10).

10. Method as claimed in any one of the preceding claims, wherein the pre-crystallization magma in the pre-crystallizers (13A, 13B, 14A, 14B, 15A, 15B, 15C) and/or the crystal suspension in the main crystallizer (10) is cooled by 0.1 to 5.0 K/h.

11. Device for obtaining a crystalline monosaccharide, in particular for carrying out the continuous method as claimed in any one of claims 1 to 10, comprising
- a main crystallizer (10) having
- means for continuously carrying out evaporation and/or cooling crystallization on a crystal suspension to generate a crystal growth of crystalline monosaccharide in the crystal suspension,
- means for separating crystals of the monosaccharide from the crystal suspension,
- a cascade for continuously forming a mass of crystallization magma for the main crystallizer, wherein the cascade comprises:
- at least one first (13) and one last (15) stage connected in series, each having at least one pre-crystallizer (13A, 13B, 15A, 15B, 15C),
- means for inoculating a solution having monosaccharide with monosaccharide seed crystals in at least one pre-crystallizer (13A, 13B) of the first stage (13) in order to obtain a pre-crystallization magma, and means for carrying out cooling crystallization and/or evaporation crystallization on the pre-crystallization magma in the at least one pre-crystallizer (13A, 13B) of the first stage (13) to form a mass of crystallization magma for the downstream stage, and
- means for supplying a solution having monosaccharide and a mass of crystallization magma from the upstream stage into the at least one pre-crystallizer (15A, 15B, 15C) of the last stage (15) in order to obtain a pre-crystallization magma, and means for carrying out cooling crystallization and/or evaporation crystallization on the pre-crystallization magma in the at least one pre-crystallizer (15A, 15B, 15C) of the last stage to form a mass of crystallization magma for the main crystallizer (10);
- means for continuously supplying a solution that contains the monosaccharide, and a mass of a crystallization magma from the at least one pre-crystallizer (15A, 15B, 15C) of the last stage (15) of the cascade into the main crystallizer (10) to form the crystal suspension.

12. Device as claimed in claim 11, wherein each stage (13, 14, 15) comprises a single pre-crystallizer (13A, 14A, 15A) and the cascade comprises means for forming a mass of crystallization magma from the pre-crystallization magma in the pre-crystallizers (13A, 14A, 15A) continuously by means of evaporation crystallization.

13. Device as claimed in claim 11, wherein each stage (13, 14, 15) comprises two to three pre-crystallizers (13A, 13B, 14A, 14B, 15A, 15B, 15C) and the cascade comprises means for discontinuously forming a mass of crystallization magma by means of cooling crystallization and/or for continuously forming a mass of crystallization magma by means of evaporation crystallization in the pre-crystallizers (13A, 13B, 14A, 14B, 15A, 15B, 15C) of each stage (13, 14, 15) from the pre-crystallization magma and, in the case of discontinuous formation of a mass of crystallization magma by means of cooling crystallization, comprises means for continuously supplying a mass of crystallization magma, alternately from the pre-crystallizers (15A, 15B, 15C) of the last stage (15), into the main crystallizer.

14. Device as claimed in any one of the preceding claims, in particular claim 11 or 13, wherein at least one stage (13, 14, 15) comprises more than one pre-crystallizer (13A, 13B, 14A, 14B, 15A, 15B, 15C) and the pre-crystallizers (13A, 13B, 14A, 14B, 15A, 15B, 15C) of the same stage each have means for forming preferably equal masses of crystallization magma.

15. Device as claimed in any one of the preceding claims, wherein the cascade between the first (13) and the last (15) stage comprises one to eight, preferably one to three, most preferably one further stage (14) or stages connected in series, wherein the further stage (14) or further stages each have at least one pre-crystallizer (14A, 14B), and the cascade has means to feed solution having monosaccharide and a mass of crystallization magma from the upstream stage (13) into the at least one pre-crystallizer (14A, 14B) of each further stage (14), in order to obtain pre-crystallization magma, and the cascade has means for forming a mass of crystallization magma in the at least one pre-crystallizer (14A, 14B) of each further stage for the downstream stage from the pre-crystallization magma discontinuously by means of cooling crystallization and/or continuously by means of evaporation crystallization.

16. Method as claimed in any one of claims 1 to 10 or device as claimed in any one of claims 11 to 15, wherein the monosaccharide is a monosaccharide, in particular a hexulose, a hexose, a pentose, or a tetrose having a melting point of 90 to 165, wherein the monosaccharide is particularly preferably a hexulose, in particular psicose (allulose), in particular D-psicose.

## Revendications

1. Procédé continu pour l'obtention d'un monosaccharide cristallin, comprenant :
- une cristallisation en continu du monosaccharide dans un cristallisoir principal (10),
- une cristallisation par évaporation et/ou par refroidissement étant effectuée en continu dans le cristallisoir principal (10) sur une suspension cristalline, afin de faire croître des cristaux de monosaccharide dans la suspension cristalline,
- une séparation des cristaux de monosaccharide de la suspension cristalline, afin d'obtenir du monosaccharide cristallin ;
- la formation en continu d'une masse de magma de cristallisation pour le cristallisoir principal (10) dans une cascade, sachant que
- la cascade comprend, montés en série, au moins un premier étage (13) et un dernier étage (15), chaque étage comprenant au moins un pré-cristallisoir (13A, 15A),
- dans ledit au moins un pré-cristallisoir (13A) du premier étage (13), une solution contenant un monosaccharide est ensemencée par des germes cristallins de monosaccharide, afin d'obtenir un magma de pré-cristallisation, et à partir du magma de pré-cristallisation, une masse de magma de cristallisation pour l'étage en aval (14, 15) est formée par cristallisation par refroidissement et/ou cristallisation par évaporation, et
- une solution contenant un monosaccharide et une masse de magma de cristallisation provenant de l'étage en amont sont introduites dans ledit au moins un pré-cristallisoir (15A, 15B, 15C) du dernier étage (15), afin d'obtenir un magma de pré-cristallisation, et, dans ledit au moins un pré-cristallisoir (15A, 15B, 15C) du dernier étage (15), une masse de magma de cristallisation pour le cristallisoir principal (10) est formée à partir du magma de pré-cristallisation par cristallisation par refroidissement et/ou cristallisation par évaporation ;
- l'introduction en continu d'une solution contenant le monosaccharide et d'une masse de magma de cristallisation, provenant dudit au moins pré-cristallisoir (15A, 15B, 15C) du dernier étage (15) de la cascade, dans le cristallisoir principal (10) afin de fournir la suspension cristalline.

2. Procédé selon la revendication 1,
dans lequel chaque étage (13, 14, 15) de la cascade comprend un seul pré-cristallisoir (13A, 14A, 15A), et, dans le pré-cristallisoir (13A, 14A, 15A) de chaque étage (13, 14, 15), une masse de magma de cristallisation est formée en continu à partir du magma de pré-cristallisation par cristallisation par évaporation.

3. Procédé selon la revendication 1,
dans lequel chaque étage (13, 14, 15) comprend deux à trois pré-cristallisoirs (13A, 13B, 14A, 14B, 15A, 15B, 15C), et, dans les pré-cristallisoirs (13A, 13B, 14A, 14B, 15A, 15B, 15C) de chaque étage (13, 14, 15), une masse de magma de cristallisation est formée à partir du magma de pré-cristallisation de manière discontinue par cristallisation par refroidissement et/ou de manière continue par cristallisation par évaporation, et, dans le cas de la formation discontinue par cristallisation par refroidissement, la masse de magma de cristallisation amenée en continu au cristallisoir principal (10) est amenée en alternance à partir des pré-cristallisoirs (15A, 15B, 15C) du dernier étage (15).

4. Procédé selon l'une des revendications précédentes, en particulier selon la revendication 1 ou 3,
dans lequel au moins un étage (13, 14, 15) comprend plus d'un pré-cristallisoir, et les pré-cristallisoirs (13A, 13B, 14A, 14B, 15A, 15B, 15C) d'un même étage (13, 14, 15) forment de préférence chacun des masses égales de magma de cristallisation.

5. Procédé selon l'une des revendications précédentes,
dans lequel la cascade entre le premier étage (13) et le dernier étage (15) comprend un à huit, de préférence un à trois, et de manière encore plus préférée un, étage(s) supplémentaire(s) (14) montés en série, chaque étage supplémentaire (14) comportant au moins un pré-cristallisoir (14A, 14B) dans lequel ou lesquels une solution contenant un monosaccharide et une masse de magma de cristallisation provenant de l'étage en amont (13) sont introduites afin d'obtenir un magma de pré-cristallisation, et, dans ledit au moins un pré-cristallisoir (14A, 14B) de chaque étage supplémentaire (14), une masse de magma de cristallisation pour l'étage en aval (15) est formée à partir du magma de pré-cristallisation de manière discontinue par cristallisation par refroidissement et/ou de manière continue par cristallisation par évaporation.

6. Procédé selon l'une des revendications précédentes,
dans lequel les germes cristallins de monosaccharide présentent un diamètre moyen de 5 à 50 µm, de préférence de 10 à 20 µm.

7. Procédé selon l'une des revendications précédentes,
dans lequel un gradient de température de la suspension cristalline sur la longueur du cristallisoir principal (10) est réglé à une température de 70 à 15 °C et de préférence de 45 à 25 °C, et/ou la durée de séjour de la suspension cristalline dans le cristallisoir principal (10) est de 30 à 70 heures.

8. Procédé selon l'une des revendications précédentes,
dans lequel le contenu de chaque pré-cristallisoir (13A, 13B, 14A, 14B, 15A, 15B, 15C), de préférence une ou plusieurs solutions, suspensions, magmas de pré-cristallisation et/ou magmas de cristallisation, est agité par un agitateur ayant une puissance spécifique de 0,1 à 4,0 kW/m³, de préférence de 0,5 à 2,0 kW/m³.

9. Procédé selon l'une des revendications précédentes,
dans lequel la solution contenant un monosaccharide pour les pré-cristallisoirs présente une sursaturation de 0 à 60 %, et/ou la solution contenant le monosaccharide est sursaturée lors de son introduction dans le cristallisoir principal (10).

10. Procédé selon l'une des revendications précédentes,
dans lequel le magma de pré-cristallisation dans les pré-cristallisoirs (13A, 13B, 14A, 14B, 15A, 15B, 15C) et/ou la suspension cristalline dans le cristallisoir principal (10) est refroidie de 0,1 à 5,0 K/h.

11. Dispositif pour l'obtention d'un monosaccharide cristallin, en particulier pour la mise en œuvre du procédé continu selon l'une des revendications 1 à 10, comprenant
- un cristallisoir principal (10) avec
- des moyens pour effectuer en continu une cristallisation par évaporation et/ou par refroidissement sur une suspension cristalline afin de provoquer la croissance de cristaux de monosaccharide cristallin dans la suspension cristalline,
- des moyens pour séparer les cristaux de monosaccharide de la suspension cristalline,
- une cascade destinée à la formation en continu d'une masse de magma de cristallisation pour le cristallisoir principal, ladite cascade comprenant :
- au moins un premier (13) et un dernier (15) étage montés en série, comportant chacun au moins un pré-cristallisoir (13A, 13B, 15A, 15B, 15C),
- des moyens pour ensemencer une solution contenant un monosaccharide avec des germes cristallins de monosaccharide dans au moins un pré-cristallisoir (13A, 13B) du premier étage (13), afin d'obtenir un magma de pré-cristallisation, et des moyens pour effectuer une cristallisation par refroidissement et/ou une cristallisation par évaporation sur le magma de pré-cristallisation dans ledit au moins un pré-cristallisoir (13A, 13B) du premier étage (13), afin de former une masse de magma de cristallisation pour l'étage en aval, et
- des moyens pour introduire une solution de monosaccharide et une masse de magma de cristallisation provenant de l'étage en amont dans ledit au moins un pré-cristallisoir (15A, 15B, 15C) du dernier étage (15), afin d'obtenir un magma de pré-cristallisation, et des moyens pour effectuer une cristallisation par refroidissement et/ou une cristallisation par évaporation sur le magma de pré-cristallisation dans ledit au moins un pré-cristallisoir (15A, 15B, 15C) du dernier étage, afin de former une masse de magma de cristallisation pour le cristallisoir principal (10) ;
- des moyens pour introduire en continu une solution contenant le monosaccharide et une masse de magma de cristallisation, provenant dudit au moins un pré-cristallisoir (15A, 15B, 15C) du dernier étage (15) de la cascade, dans le cristallisoir principal (10) afin de former la suspension cristalline.

12. Dispositif selon la revendication 11,
dans lequel chaque étage (13, 14, 15) comprend un seul pré-cristallisoir (13A, 14A, 15A), et la cascade comprend des moyens pour former en continu une masse de magma de cristallisation à partir du magma de pré-cristallisation dans les pré-cristallisoirs (13A, 14A, 15A) par cristallisation par évaporation.

13. Dispositif selon la revendication 11,
dans lequel chaque étage (13, 14, 15) comprend deux à trois pré-cristallisoirs (13A, 13B, 14A, 14B, 15A, 15B, 15C), et la cascade comprend des moyens pour former de manière discontinue une masse de magma de cristallisation par cristallisation par refroidissement et/ou pour former de manière continue une masse de magma de cristallisation par cristallisation par évaporation dans les pré-cristallisoirs (13A, 13B, 14A, 14B, 15A, 15B, 15C) de chaque étage (13, 14, 15) à partir du magma de pré-cristallisation, ainsi que, dans le cas d'une formation discontinue d'une masse de magma de cristallisation par cristallisation par refroidissement, des moyens pour amener en continu une masse de magma de cristallisation, provenant en alternance des pré-cristallisoirs (15A, 15B, 15C) du dernier étage (15), au cristallisoir principal.

14. Dispositif selon l'une des revendications précédentes, en particulier selon la revendication 11 ou 13,
dans lequel au moins un étage (13, 14, 15) comprend plus d'un pré-cristallisoir (13A, 13B, 14A, 14B, 15A, 15B, 15C), et les pré-cristallisoirs (13A, 13B, 14A, 14B, 15A, 15B, 15C) d'un même étage comportent chacun des moyens pour former de préférence des masses égales de magma de cristallisation.

15. Dispositif selon l'une des revendications précédentes,
dans lequel la cascade entre le premier (13) et le dernier (15) étage comprend un à huit, de préférence un à trois, et de manière encore plus préférée un, étage(s) supplémentaire(s) (14) montés en série, lesdits étages supplémentaires (14) comprenant chacun au moins un pré-cristallisoir (14A, 14B), et la cascade comprend des moyens pour introduire dans ledit au moins un pré-cristallisoir (14A, 14B) de chaque étage supplémentaire (14) une solution contenant un monosaccharide et une masse de magma de cristallisation provenant de l'étage en amont (13), afin d'obtenir un magma de pré-cristallisation, et la cascade comprend des moyens pour former une masse de magma de cristallisation dans ledit au moins un pré-cristallisoir (14A, 14B) de chaque étage supplémentaire, pour l'étage en aval, à partir du magma de pré-cristallisation, de manière discontinue par cristallisation par refroidissement et/ou de manière continue par cristallisation par évaporation.

16. Procédé selon l'une des revendications 1 à 10 ou dispositif selon l'une des revendications 11 à 15,
dans lequel le monosaccharide est un monosaccharide, en particulier un hexulose, un hexose, un pentose ou un tétrose, ayant un point de fusion compris entre 90 et 165 °C, le monosaccharide étant de préférence un hexulose, en particulier un psicose (allulose), et plus particulièrement un D-psicose.
